# EUROPEAN PATENT APPLICATION

(11) **EP 3 326 565 A1**
(43) Date of publication of application: **30.05.2018**
(21) Application number: 16827861.2
(22) Date of filing: 22.07.2016
(51) Int. Cl.: A61B 34/35, B25J 3/00

(54) **INPUT MECHANISM AND MEDICAL SYSTEM**

(30) Priority: 23.07.2015 US 201562195869 P
(71) Applicant: Olympus Corporation, Hachioji-shi, Tokyo 192-8507 (JP)
(72) Inventor: HARAGUCHI Masafumi, Hachioji-shi Tokyo 192-8507 (JP)
(74) Representative: Gunzelmann, Rainer
(86) International application number: PCT/JP2016/071581
(87) International publication number: WO 2017/014301

(57) **Abstract**

An input mechanism (31) includes a first pivoting section (33) that is pivotable with a first axis (X) as a rotational center, a second pivoting section (35) that is pivotable with respect to the first pivoting section (33) with a second axis (Y) as a rotational center crossing the first axis (X), a first link (34) fixed to the first pivoting section (33) and connected to the second pivoting section (35) such that the second pivoting section (35) is pivotable with the second axis (Y), a second link (36) fixed to the second pivoting section (35), an advance/retreat input section (37) connected to the second link (36) and thereby enable an advance/retreat operation along a third axis (L) passing through an intersection between the first axis (X) and the second axis (Y) as an advance/retreat axis, and a sensor unit (40) configured to determine a rotation angle of the first axis (X), a rotation angle of the second axis (Y) and an advance/retreat movement distance of the advance/retreat input section (37).

## Description

### [Technical Field]

The present invention relates to an input mechanism configured to input an operation to a medical system, and a medical system including the input mechanism.

Priority is claimed on United States Patent Provisional Application No. 62/195,869, filed July 23, 2015, the content of which is incorporated herein by reference.

### [Background Art]

Medical systems for performing surgery or the like in the body are widely known.

For example, Patent Document 1 discloses a medical manipulator configured to change an orientation of a treatment section for treating a patient with two degrees of freedom or more. In the medical manipulator disclosed in Patent Document 1, the treatment section is connected to an operation instructing section configured to operate the treatment section by a rod-shaped connecting section. The treatment section of the medical manipulator disclosed in Patent Document 1 can change an orientation with two degrees of freedom or more according to an operation of an operation instructing section. Further, the treatment section of the medical manipulator disclosed in Patent Document 1 can move by swinging the connecting section about a predetermined point set on the connecting section as a swinging center.

### [Citation List]

### [Patent Literature]

### [Patent Document 1]

Japanese Unexamined Patent Application, First Publication No. 2007-260431

### [Summary of Invention]

### [Technical Problem]

In the technology disclosed in Patent Document 1, when a direction in which the treatment section approaches a treatment object area is to be changed, a combination of an operation of directing the treatment section toward the treatment object area and an operation of adjusting a position of the treatment section with respect to the treatment object area is required, and the operations are complicated.

In consideration of the above-mentioned circumstances, the present invention is to provide an input mechanism and a medical system with which an operation of changing a direction in which a treatment object area is approached can be intuitively performed.

### [Solution to Problem]

An aspect of the present invention is an input mechanism installed in a medical system having a manipulator and configured to input an operation to the manipulator, the input mechanism including: a first pivoting section that is pivotable with a first axis as a rotational center; a second pivoting section that is pivotable with respect to the first pivoting section with a second axis crossing the first axis as a rotational center; a first link fixed to the first pivoting section and connected to the second pivoting section such that the second pivoting section is pivotable with the second axis, a second link fixed to the second pivoting section; an advance/retreat input section connected to the second link and thereby enable an advance/retreat operation along a third axis passing through an intersection between the first axis and the second axis as an advance/retreat axis; and a sensor unit configured to determine a rotation angle of the first axis, a rotation angle of the second axis and an advance/retreat movement distance of the advance/retreat input section.

The first axis and the second axis may be perpendicular to each other.

The input mechanism of the above-mentioned aspect may have a first locking mechanism configured to prohibit pivotal movement with the first axis and the second axis.

The input mechanism of the above-mentioned aspect may have a second locking mechanism configured to prohibit an advance/retreat movement of the advance/retreat input section along the third axis.

The first link may be constituted by a rigid body bent in a "<" shape.

The second link may be constituted by a rigid body bent in a "<" shape.

Another aspect of the present invention is a medical system including the input mechanism of the above-mentioned aspect, a control unit connected to the sensor unit; a manipulator connected to the control unit; and a suspension apparatus configured to hold the manipulator, wherein the manipulator has: an imaging unit configured to image a treatment object area; an active bending section connected to the imaging unit; a shaft section connected to the active bending section; and a first driving unit connected to the shaft section and configured to bend the active bending section in accordance with control by the control unit, the suspension apparatus has: an arm section connected to the manipulator; and a second driving unit connected to the arm section and configured to operate the arm section in accordance with control by the control unit, and the control unit includes: a constraint point coordinate computing unit configured to set a constraint point that is to be a swinging center of the manipulator in a predetermined reference coordinate system, the reference coordinate system being set in the suspension apparatus in a state in which the manipulator is attached; a position-of-interest coordinate setting unit configured to set a position of interest in an imaging field of vision of the imaging unit on the basis of an image imaged by the imaging unit; a coordinate converting unit configured to associate an input coordinate system preset in the input mechanism with the reference coordinate system such that an intersection between the first axis and the second axis corresponds to coordinates of the position of interest in the reference coordinate system; an operation instruction generating unit configured to allow the imaging unit to move according to a rotation angle of the first axis, a rotation angle of the second axis and an advance/retreat movement distance of the advance/retreat input section and calculate operation amounts of the arm section and the active bending section on the basis of a detection state in the sensor unit such that the constraint point does not move in the reference coordinate system; and a driving signal generating unit configured to operate the first driving unit and the second driving unit on the basis of the operation amount.

The control unit may further include a constraint point coordinate computing unit configured to set a constraint point that is to be a swinging center of the manipulator in the reference coordinate system, and the operation instruction generating unit may calculate the operation amount such that the constraint point does not move in the reference coordinate system.

The medical system of the above-mentioned aspect may further include a mode selector connected to the control unit to receive an input in order to switch between a first operation mode of operating the first driving unit and the second driving unit by using the position-of-interest coordinate setting unit, the coordinate converting unit, the operation instruction generating unit and the driving signal generating unit, and a second operation mode of allowing an operator to directly operate the manipulator.

### [Advantageous Effects of Invention]

According to the present invention, an operation of changing a direction in which a treatment object area is approached can be intuitively performed.

### [Brief Description of Drawings]

Fig. 1 is a general view of a medical system including an input mechanism of a first embodiment of the present invention.
Fig. 2 is a schematic view of a manipulator of the medical system.
Fig. 3 is a perspective view showing an input mechanism of an operation input apparatus of the medical system.
Fig. 4 is a block diagram schematically showing the medical system as a whole.
Fig. 5 is a block diagram showing major parts of the medical system.
Fig. 6 is a block diagram showing parts of a control unit and an operation input apparatus of the medical system.
Fig. 7 is a table showing a brake control pattern of the medical system.
Fig. 8 is a table showing the brake control pattern of the medical system.
Fig. 9 is a block diagram of major parts of the medical system.
Fig. 10 is a block diagram of major parts of the medical system.
Fig. 11 is a block diagram of major parts of the medical system.
Fig. 12 is a block diagram of major parts of the medical system.
Fig. 13 is a block diagram of major parts of the medical system.
Fig. 14 is a flowchart showing an operation sequence of an operator who operates the medical system.
Fig. 15 is a flowchart showing major parts of a method of actuating a medical system.
Fig. 16 is a view showing an action of the medical system.
Fig. 17 is a view showing the action of the medical system.
Fig. 18 is a view showing the action of the medical system.
Fig. 19 is a perspective view showing an input mechanism according to a variant of the embodiment.
Fig. 20 is a perspective view showing an input mechanism according to another variant of the embodiment.
Fig. 21 is a perspective view showing the input mechanism according to the other variant of the embodiment.
Fig. 22 is a view for describing a variation in positional relation between a first link and a second link in an input mechanism of still another variant of the embodiment.
Fig. 23 is a block diagram showing a major part of a medical system including an input mechanism of a second embodiment of the present invention.
Fig. 24 is a block diagram showing the major parts of the medical system.
Fig. 25 is a table showing a brake control pattern of the medical system.
Fig. 26 is a block diagram showing the major parts of the medical system.
Fig. 27 is a block diagram showing the major parts of the medical system.
Fig. 28 is a flowchart showing major parts of a method of actuating a medical system.

### [Description of Embodiments]

### (First embodiment)

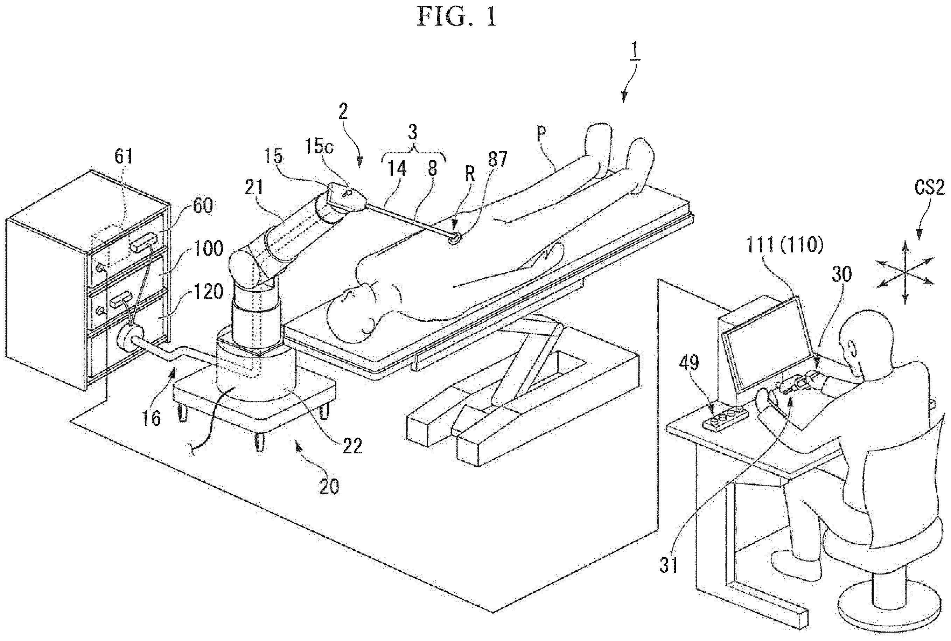

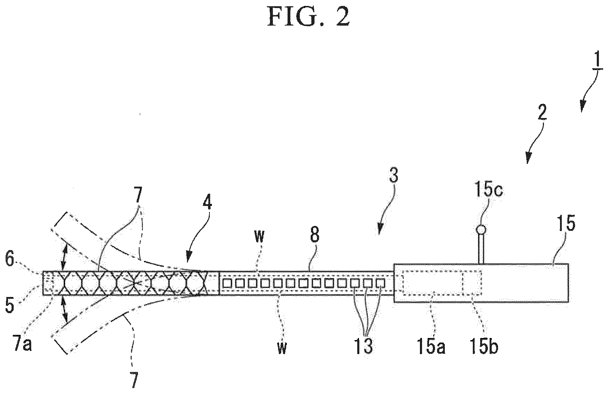

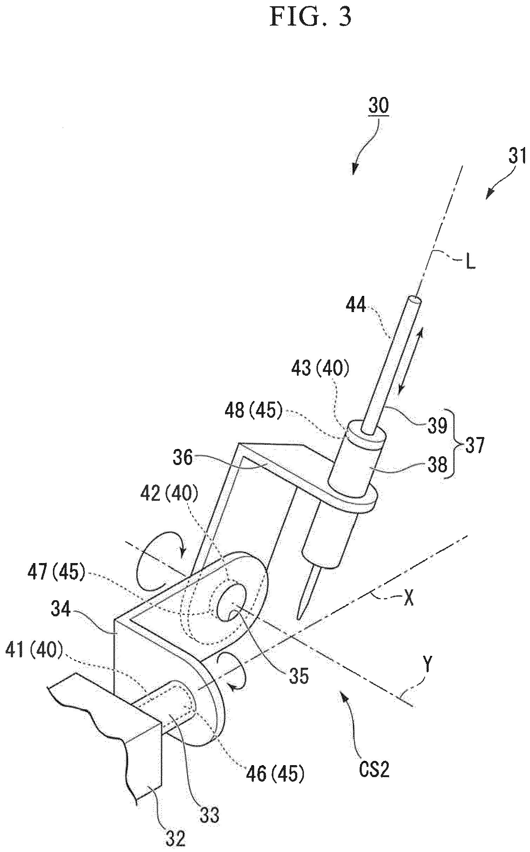

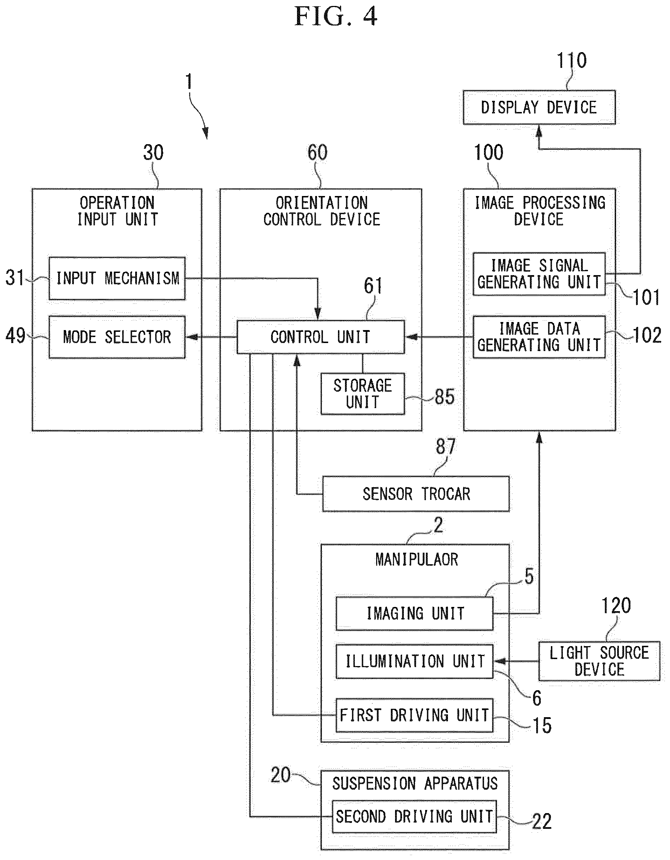

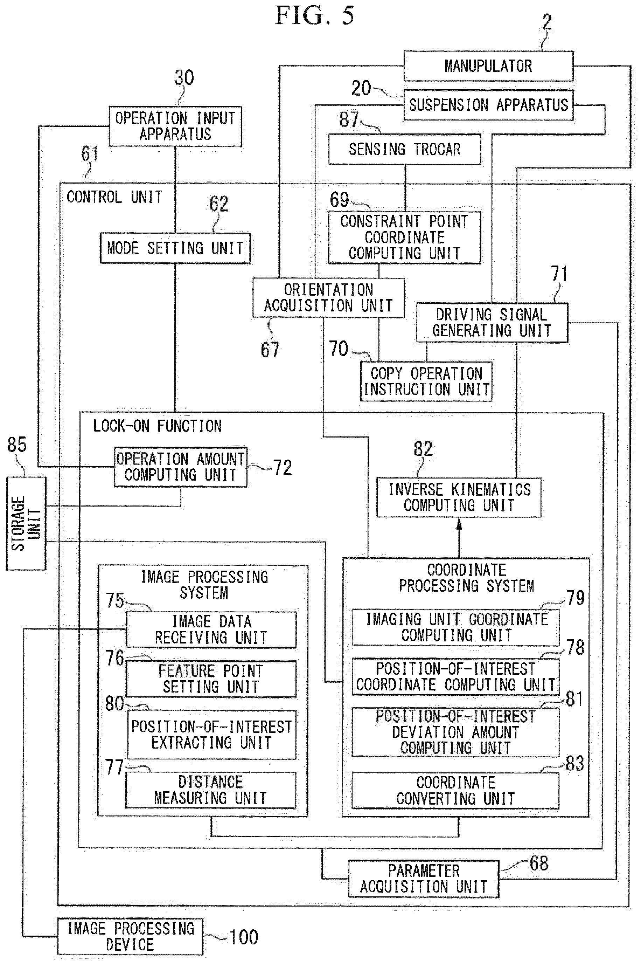

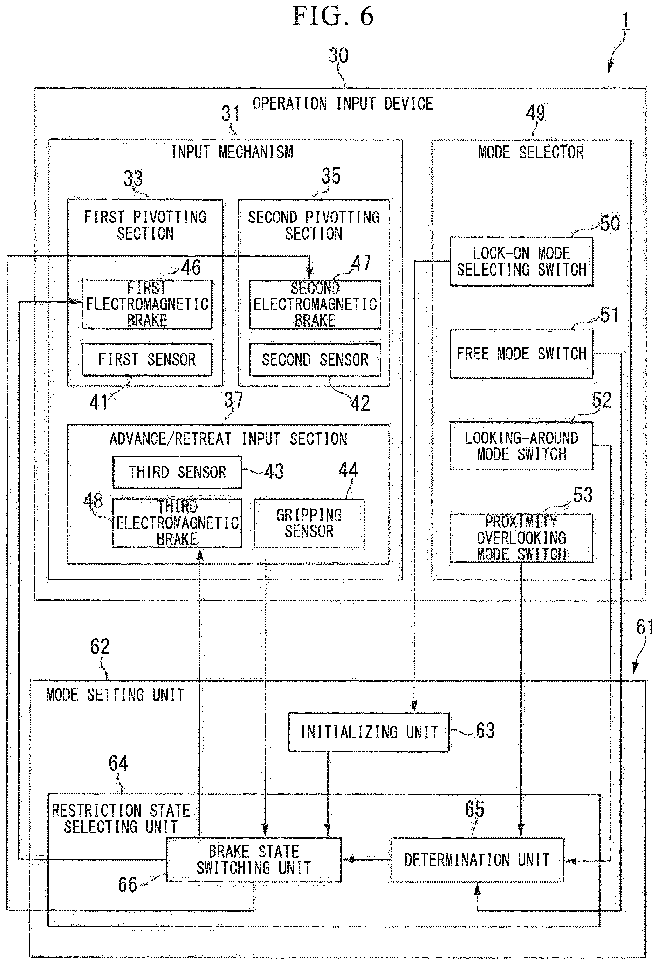

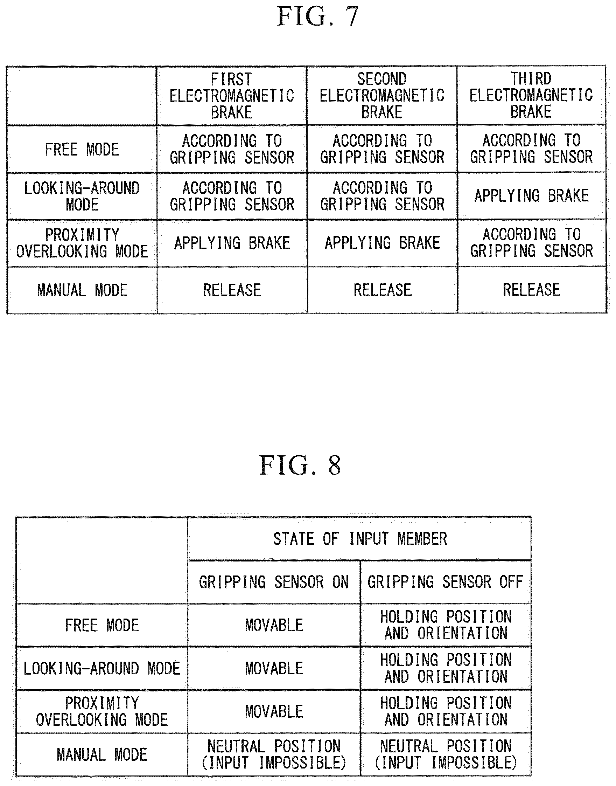

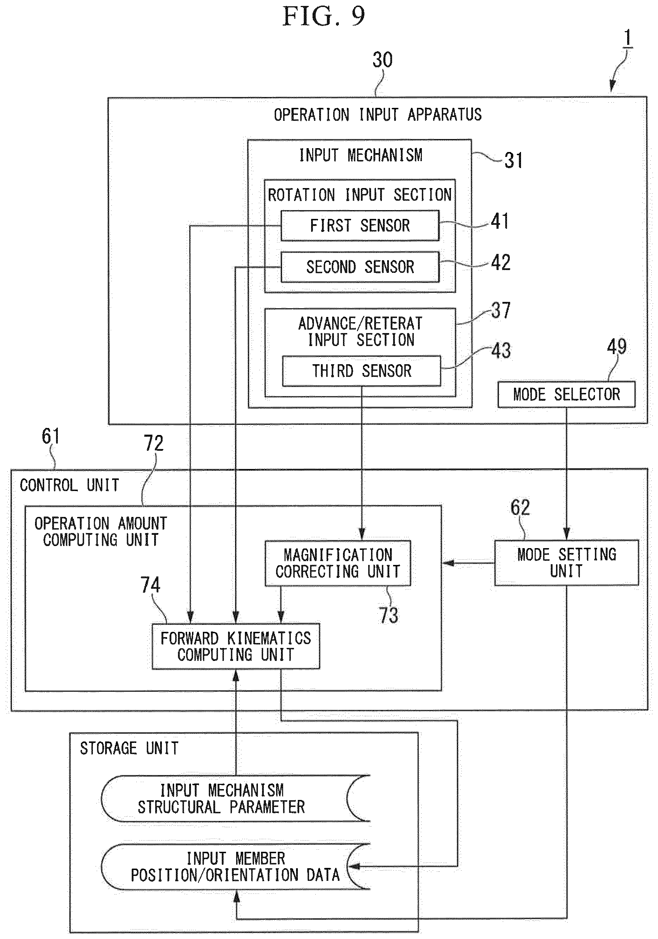

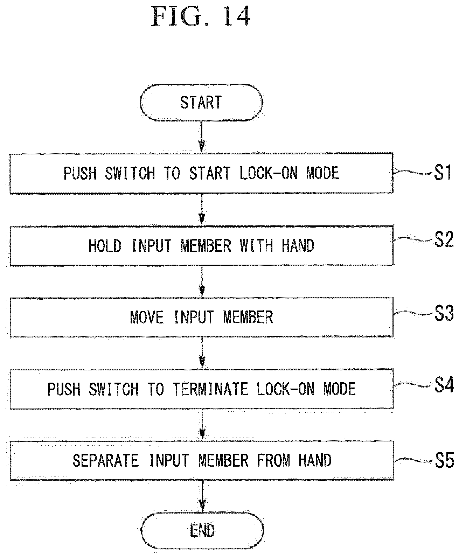

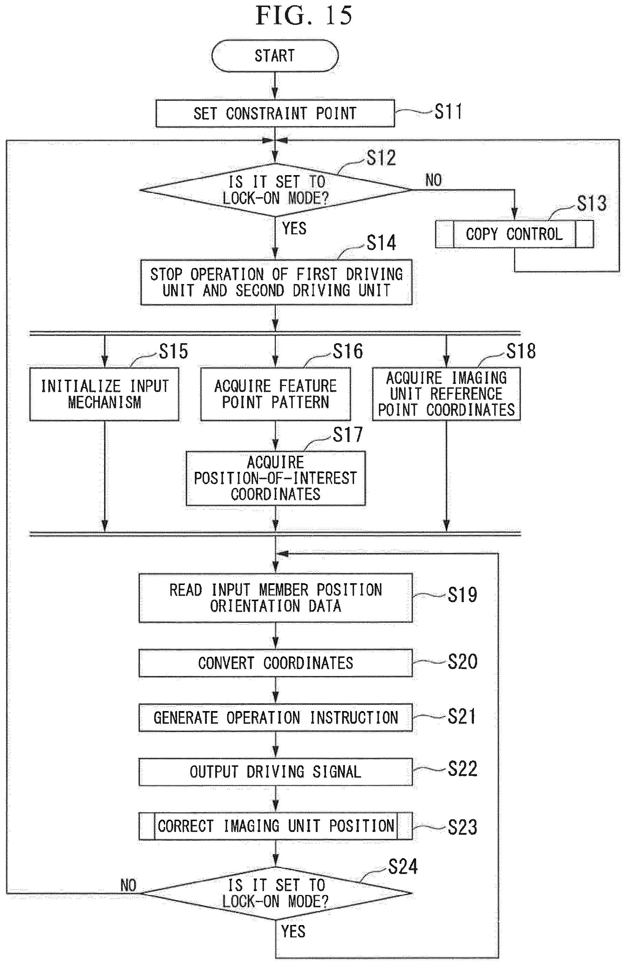

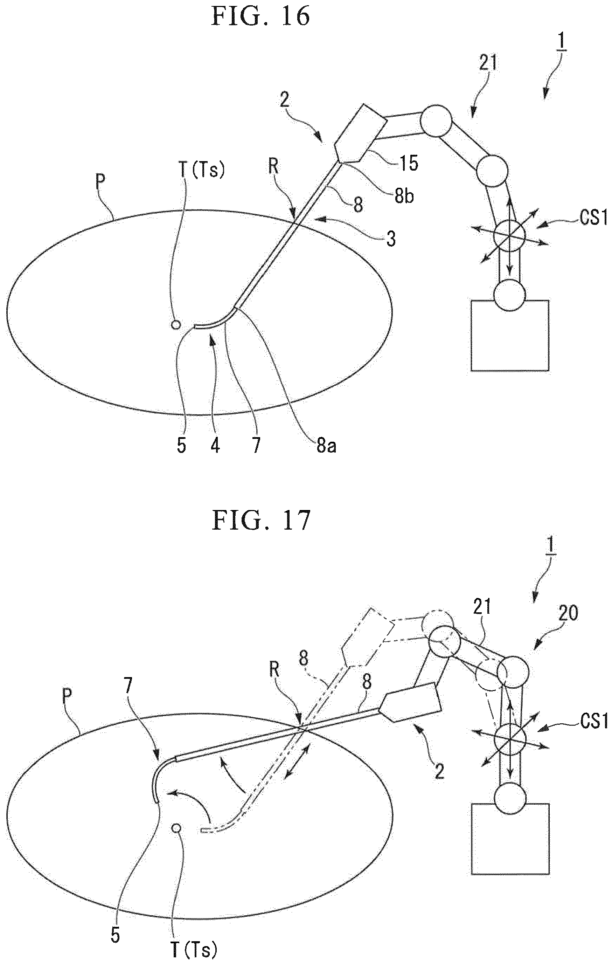

A first embodiment of the present invention will be described. Fig. 1 is a general view of a medical system including an input mechanism of the embodiment. Fig. 2 is a schematic view showing a manipulator of the medical system. Fig. 3 is a perspective view showing the input mechanism of an operation input apparatus of the medical system. Fig. 4 is a block diagram schematically showing the medical system as a whole. Fig. 5 is a block diagram showing major parts of the medical system. Fig. 6 is a block diagram showing parts of a control unit and an operation input apparatus of the medical system. Figs. 7 and 8 are tables showing brake control patterns of the medical system. Figs. 9 to 13 are block diagrams of major parts of the medical system. Fig. 14 is a flowchart showing an operation sequence of an operator who operates the medical system. Fig. 15 is a flowchart showing major parts of a method of actuating a medical system. Figs. 16 to 18 are views showing actions of the medical system.

As shown in Fig. 1, a medical system 1 of the embodiment has a manipulator 2, a suspension apparatus 20, an operation input apparatus 30, an orientation control device 60 and a sensing trocar 87. In addition, the medical system 1 of the embodiment may include an image processing device 100, a display device 110 and a light source device 120, which are known.

The manipulator 2 shown in Figs. 1 and 2 is an apparatus inserted into a body cavity of a patient P and is configured to perform observation of an organ or the like or treatment of the organ or the like (see Fig. 16). As shown in Fig. 1, the manipulator 2 is connected to the suspension apparatus 20, the operation input apparatus 30, the orientation control device 60, the image processing device 100, the display device 110 and the light source device 120.

The manipulator 2 has an insertion section 3, a driving unit (a first driving unit 15) and a connecting section 16.

As shown in Figs. 1 and 2, the insertion section 3 is an elongatedmember that can be used when inserted into the body cavity. The insertion section 3 has a distal end portion 4, a shaft section 8, an insertion amount marker 13 and a proximal end portion 14. The distal end portion 4 and the shaft section 8 can be inserted into the body cavity. The distal end portion 4 is disposed on a distal side of the shaft section 8 in an insertion direction in which it is inserted into the body cavity. The proximal end portion 14 is disposed on a base end side of the shaft section 8.

The distal end portion 4 of the insertion section 3 has an imaging unit 5, an illumination unit 6 and an active bending section 7. The active bending section 7 is connected to a distal part of the shaft section 8. The bent shape of the active bending section 7 can be actively changed, which will be described below. In addition, the imaging unit 5 and the illumination unit 6 are disposed closer to a distal side than the active bending section 7. Accordingly, the imaging unit 5 and the illumination unit 6 can change positions and directions thereof according to the bent shape of the active bending section 7.

The imaging unit 5 is an end effector configured to acquire an image of a target that is a treatment object. An optical axis of the imaging unit 5 extends from the most distal end of the distal end portion 4 toward a front side of the distal end portion 4. For example, the optical axis of the imaging unit 5 extends parallel to a longitudinal axis of the distal end portion 4 of the insertion section 3. Accordingly, the imaging unit 5 of the embodiment has an imaging field of vision in a region in front of the distal end portion 4 of the insertion section 3. A structure of an observation means in a known endoscope, for example, a CCD image pickup element or the like, may be appropriately selected and employed as the imaging unit 5.

The imaging unit 5 can image an image to measure a distance to an object captured in a field of vision. For example, the imaging unit 5 includes a set of image pickup elements that can image a set of images (a first image and a second image) having parallax. Further, instead of the imaging unit 5 including the set of image pickup elements, the imaging unit 5 may include an optical system in which a set of images having parallax are imaged on one image pickup element. The set of images imaged by the imaging unit 5 are used to measure a distance by stereoscopic analysis in a control unit 61, which will be described below. Further, the imaging unit 5 may not have a function of acquiring an image for measuring a distance. In this case, a known range finding mechanism such as a laser range finder or the like is disposed on the distal end portion 4 of the insertion section 3.

The imaging unit 5 outputs signals of a set of acquired images (image signals) to the image processing device 100 through the connecting section 16, which will be described below.

The illumination unit 6 illuminates an imaging field of vision of the imaging unit 5 using light transmitted from the light source device 120 through an optical fiber (not shown). Further, when the light source device 120 is not provided, the illumination unit 6 may have a light emitting element such as an LED or the like configured to emit illumination light toward a front side of the distal end portion 4.

The active bending section 7 has a plurality of joint rings 7a (bending pieces) that form a tubular shape.

The plurality of joint rings 7a are arranged in a row. Two neighboring joint rings 7a among the plurality of joint rings 7a are connected to be bendable using an axis perpendicular to a centerline of the joint ring as a bending axis.

The joint ring 7a disposed closest to the distal side of the active bending section 7 among the plurality of joint rings 7a is connected to a distal end of an angle wire w configured to operate the active bending section 7 in a bent shape. In the embodiment, four angle wires w are disposed around a centerline of the joint rings 7a at 90° intervals to bend the active bending section 7 in four directions, that is, up, down, left and right. Accordingly, the active bending section 7 can bend and deform to a desired bending quantity in a desired direction while the joint rings 7a are bent by selectively pulling the four angle wires w.

The shaft section 8 is constituted by a hard tubular member. In addition, the angle wire w that connects the active bending section 7 and the first driving unit 15 is inserted through the shaft section 8. The shaft section 8 is in communication with the distal end portion 4 and the proximal end portion 14 of the insertion section 3.

The insertion amount marker 13 includes a plurality of markers that can be detected by the sensing trocar 87. For example, in the embodiment, a linear encoder configured to determine the advance/retreat quantity of the shaft section 8 in a centerline direction of the shaft section 8 is constituted by the insertion amount marker 13 and the sensing trocar 87. The configuration of the insertion amount marker 13 may be appropriately selected according to the configuration of the sensing trocar 87.

Instead of the configuration including the insertion amount marker 13 and the sensing trocar 87, a configuration (a means) of determining a position and an orientation of the manipulator 2 may be provided. In this case, an advance/retreat quantity of the shaft section 8 in the centerline direction of the shaft section 8 can be calculated from the values of the determined position and orientation.

The first driving unit 15 is connected to the proximal end portion 14 of the insertion section 3. The first driving unit 15 has four actuators (for example, motors) 15a configured to emit power for individually pulling the four angle wires w, pulleys (not shown) connected to output shafts of the actuators 15a to transmit the power transmitted from the actuators 15a to the angle wires w, and encoders 15b configured to determine the operation amounts of the actuators 15a.

The power transmitted from the first driving unit 15 is transmitted to the active bending section 7 through the above-mentioned angle wires. The first driving unit 15 of the embodiment has a lever 15c or the like configured to allow an operator to designate a direction in which the active bending section 7 is bent or a bending quantity to operate the first driving unit 15.

The connecting section 16 shown in Fig. 1 is connected to a driving unit 15. The connecting section 16 electrically connects the manipulator 2 to the operation input apparatus 30, the orientation control device 60 and the image processing device 100.

The connecting section 16 has a signal line configured to output a signal of an image (an image signal) imaged by the imaging unit 5 to the image processing device 100, a signal line configured to output a driving signal to the first driving unit 15 of the manipulator 2 from the control unit 61, which will be described below, a signal line configured to output a driving signal to a second driving unit 22 of a suspension apparatus from the control unit 61, which will be described below, and a signal line configured to output angle information determined by an encoder (not shown) of the manipulator 2 to the control unit 61, which will be described below.

The connecting section 16 has an optical fiber configured to transmit illumination light from the light source device 120 to the manipulator 2.

As shown in Fig. 1, the suspension apparatus 20 has an arm section 21 connected to the first driving unit 15 of the manipulator 2, and a driving unit (the second driving unit 22) configured to drive the arm section 21. The arm section 21 has at least three degrees of freedom.

The second driving unit 22 is connected to the orientation control device 60. As the suspension apparatus 20 of the embodiment, a configuration of a known electric holder capable of holding and operating a hard mirror may be appropriately selected and employed. The second driving unit 22 has an actuator 22a configured to drive the arm section 21 according to a driving signal from the orientation control device 60, and an encoder 22b configured to output an operation amount of the actuator 22a to the orientation control device 60.

As shown in Figs. 1 and 4, the operation input apparatus 30 has an input mechanism 31 and a mode selector 49.

As shown in Fig. 3, the input mechanism 31 has a base section 32, a first pivoting section 33, a first link 34, a second pivoting section 35, a second link 36, an advance/retreat input section 37, a sensor unit 40 and a brake unit 45.

The first pivoting section 33 is connected to the base section 32 to be pivotable with respect to the base section 32 with a predetermined first axis X as a rotational center. The first pivoting section 33 has a neutral mechanism (not shown) such as a spring or the like that returns to a predetermined neutral position when a first brake 46 (to be described below) is in a release state while no external force is applied to the first pivoting section 33.

The first link 34 is fixed to the first pivoting section 33. The first link 34 is constituted by a rigid body having a shape bent in a "<" shape (an L shape). As an example, the first link 34 extends from the first pivoting section 33 in a direction perpendicular to the first axis X, is bent 90° away from the first pivoting section 33, and extends parallel to the first axis X from the bent area. An end of the first link 34 opposite to an end to which the first pivoting section 33 is fixed is connected to the second pivoting section 35.

The second pivoting section 35 is connected to the first link 34 to be pivotable with respect to the first link 34 with a second axis Y perpendicular to the first axis X as a rotational center. The second pivoting section 35 has a neutral mechanism (not shown) such as a spring or the like that returns it to a predetermined neutral position when a second brake 47 (to be described below) is in a release state while no external force is applied to the second pivoting section 35.

The second link 36 is fixed to the second pivoting section 35. The second link 36 is constituted by a rigid body bent in a "<" shape (an L shape). As an example, the second link 36 extends from the second pivoting section 35 in a direction perpendicular to the second axis Y and extends to be bent 90° to be parallel to the second axis Y. An end of the second link 36 opposite to an end to which the second pivoting section 35 is fixed is connected to the advance/retreat input section 37.

The advance/retreat input section 37 has a holding section 38 fixed to the second link 36, and an input member 39 connected to the holding section 38.

The holding section 38 is a tubular member having a centerline that is a straight line passing an intersection between the first axis X and the second axis Y.

The input member 39 is a rod-shaped member inserted through the holding section 38. The input member 39 can freely advance and retreat along a centerline of the holding section 38. The input member 39 can advance and retreat along an advance/retreat axis that is a third axis L having a straight line shape passing an intersection between the first axis X and the second axis Y. A shape of the input member 39 may be appropriately determined in consideration of ease of holding by an operator.

The advance/retreat input section 37 has a neutral mechanism (not shown) such as a spring or the like that returns it to a predetermined neutral position when the third brake 48 (to be described below) is in a release state while no external force is applied to the advance/retreat input section 37.

In the embodiment, movement of the input member 39 is limited to swinging about the intersection between the first axis X and the second axis Y and advance/retreat along a straight line toward the intersection between the first axis X and the second axis Y by the base section 32, the first pivoting section 33, the first link 34, the second pivoting section 35, the second link 36 and the advance/retreat input section 37.

The sensor unit 40 has a first sensor 41 disposed on the first pivoting section 33 and configured to determine a rotation angle of the first pivoting section 33 with respect to the base section 32, a second sensor 42 disposed on the second pivoting section 35 and configured to determine a rotation angle of the second pivoting section 35 with respect to the first link 34, a third sensor 43 disposed on the advance/retreat input section 37 and configured to determine an advance/retreat moving quantity of the input member 39 with respect to the holding section 38, and a gripping sensor 44 disposed on the input member 39 and configured to determine whether an operator grips the input member 39 by detecting the presence of contact of the operator with the input member 39.

As shown in Fig. 9, the first sensor 41, the second sensor 42 and the third sensor 43 are connected to an operation amount computing unit 72 of the control unit 61.

As shown in Fig. 6, the gripping sensor 44 is connected to a mode setting unit 62 of the control unit 61.

As shown in Fig. 3, the brake unit 45 has the first brake 46 disposed on the first pivoting section 33, the second brake 47 disposed on the second pivoting section 35, and a third brake 48 disposed on the advance/retreat input section 37. The first brake 46, the second brake 47 and the third brake 48 are constituted by, for example, electromagnetic brakes.

The first brake 46 switches between a state in which the first pivoting section 33 is pivotable with respect to the base section 32 and a state in which the first pivoting section 33 is not pivotable with respect to the base section 32 according to control by the orientation control device 60.

The second brake 47 switches between a state in which the second pivoting section 35 is pivotable with respect to the first link 34 and a state in which the second pivoting section 35 is pivotable with respect to the first link 34 according to control by the orientation control device 60.

The third brake 48 switches between a state in which the input member 39 is able to advance and retreat with respect to the holding section 38 and a state in which the input member 39 is unable to advance and retreat with respect to the holding section 38 according to control by the orientation control device 60.

As shown in Figs. 4 and 6, the mode selector 49 is connected to the orientation control device 60. The mode selector 49 has a plurality of switches (a lock-on mode selecting switch 50, a free mode switch 51, an inspection mode switch 52 and a proximity overviewing mode switch 53) configured to select an operation mode of the orientation control device 60.

The lock-on mode selecting switch 50 is a switch configured to switch between a lock-on mode and a manual mode. The lock-on mode is a first operation mode set in the medical system 1 to automatically operate the manipulator 2 such that the imaging unit 5 is normally directed to a position of interest in an imaging field of vision of the imaging unit 5 of the manipulator 2. The lock-on mode will be described below in detail. In addition, the manual mode is a second operation mode set in the medical system 1 to allow an operator to directly operate the manipulator 2 or move an imaging field of vision of the imaging unit 5 in a desired direction. Whenever the lock-on mode selecting switch 50 is pressed, the lock-on mode selecting switch 50 outputs a switching signal to alternately switch an operation mode of the orientation control device 60 to the lock-on mode or the manual mode.

Figs. 7 and 8 show the brake control patterns of the brake unit 45 in the lock-on mode and the manual mode.

As shown in Figs. 7 and 8, the free mode switch 51 is a switch configured to operates the orientation control device 60 in "a free mode" in which the brake unit 45 is controlled so as to release all of the brakes of the brake unit 45 while an operator grips the input member 39 (see Fig. 3) and operate all of the brakes of the brake unit 45 when the operator has released the input member 39 in the lock-on mode. That is, in the free mode, the operator can freely operate the input member 39 when the operator holds the input member 39 with his/her hand, and the position and the orientation of the input member 39 are held instantly when the operator releases his/her hand from the input member 39. Whenever the free mode switch 51 is pressed, the free mode switch 51 outputs a free mode selection signal to switch an operation mode of the orientation control device 60 to a free mode.

The inspection mode switch 52 is a switch configured to operates the orientation control device 60 in "an inspection mode" in which the brake unit 45 is controlled so as to operate the third brake 48 normally, release the other brakes (the first brake 46 and the second brake 47) while the operator grips the input member 39, and operate all of the brakes of the brake unit 45 when the operator releases the input member 39 in the lock-on mode. That is, in the inspection mode, while the operator can freely swing the input member 39 while the operator holds the input member 39 with his/her hand, the input member 39 cannot be advanced and retreated. In addition, in the inspection mode, the position and the orientation of the input member 39 are held instantly when the operator releases his/her hand from the input member 39. Whenever the inspection mode switch 52 is pressed, the inspection mode switch 52 outputs an inspection mode selection signal to switch the operation mode of the orientation control device 60 to the inspection mode. In the embodiment, when the inspection mode is selected, the control unit 61 and the brake unit 45 are configured as a first locking mechanism configured to prohibit pivotal movement on the first axis X and the second axis Y.

The proximity overviewing mode switch 53 is a switch configured to operate the orientation control device 60 in "a proximity overviewing mode" in which the brake unit 45 is controlled so as to operate the first brake 46 and the second brake 47 normally, release the third brake 48 while the operator grips the input member 39, and operate all of the brakes of the brake unit 45 when the operator releases the input member 39 in the lock-on mode. That is, in the proximity overviewing mode, while the operator can freely advance and retreat the input member 39 while the operator holds the input member 39 with his/her hand, the input member 39 cannot be swung. In addition, in the proximity overviewing mode, the position and the orientation of the input member 39 are held instantly when the operator releases his/her hand from the input member 39. Whenever the proximity overviewing mode switch 53 is pressed, the proximity overviewing mode switch 53 outputs a proximity overviewing mode selection signal to switch the operation mode of the orientation control device 60 to the proximity overviewing mode. In the embodiment, when the proximity overviewing mode is selected, the control unit 61 and the brake unit 45 are configured as a second locking mechanism configured to prohibit advance/retreat of the input member 39 on the third axis L.

The free mode switch 51, the inspection mode switch 52 and the proximity overviewing mode switch 53 (see Fig. 5) are linked such that the other switches are automatically turned OFF when one switch is pushed ON. For this reason, in the mode selector 49, any one of the free mode, the inspection mode and the proximity overviewing mode is selected in the lock-on mode.

In the embodiment, when the operation mode is set to the manual mode according to the operation of the lock-on mode selecting switch 50, all of the brakes are released and the input member 39 is returned to the neutral position. In addition, when the operation mode is set to the manual mode, the free mode switch 51, the inspection mode switch 52 and the proximity overviewing mode switch 53 are turned OFF.

As shown in Fig. 4, the image processing device 100 receives an input of the image signal output from the imaging unit 5. The image processing device 100 has a video signal generating unit 101 configured to encode the image signal as a video signal. The video signal generating unit 101 converts one predetermined image (for example, in the embodiment, a first image) of a set of images (a first image and a second image) included in the image signal into a video signal having a format appropriate for display on the display device 110 on the basis of the image signal output from the imaging unit 5. The image processing device 100 outputs the video signal generated by the video signal generating unit 101 to the display device 110.

The image processing device 100 has an image data generating unit 102 configured to encode the image signal as image data. The image data generating unit 102 encodes the signal of the first image included in the image signal to first image data and encodes the signal of the second image included in the image signal as second image data on the basis of the image signal output from the imaging unit 5. The image processing device 100 outputs a set of image data including the first image data and the second image data to the control unit 61.

The display device 110 receives an input of the video signal output from the video signal generating unit 101 of the image processing device 100. The display device 110 has, for example, a liquid crystal monitor 111.

The orientation control device 60 shown in Fig. 1 can be connected to the manipulator 2, the suspension apparatus 20, the operation input apparatus 30 and the image processing device 100. The orientation control device 60 has the control unit 61 configured to control the positions and the orientations of the manipulator 2 and the suspension apparatus 20, and a storage unit 85 configured to store data generated by the control unit 61, parameters referred to by the control unit 61, or the like.

As shown in Fig. 9, the parameters stored in the storage unit 85 of the orientation control device 60 include manipulator structural parameters corresponding to a configuration of the manipulator 2, and suspension apparatus structural parameters corresponding to a configuration of the suspension apparatus 20. These parameters include data such as a distance or the like between a joint and a joint coordinate system that can be used for forward kinematics calculation and inverse kinematics calculation. In addition, in the embodiment, for the manipulator structural parameters, a joint coordinate system is determined with respect to an imaging unit reference point (not shown) defined at one place of the imaging unit 5 of the manipulator 2. A distance value acquired by measuring the distance to an object imaged by the imaging unit 5 is calculated on the basis of the distance from the imaging unit reference point to the object.

As shown in Fig. 5, the control unit 61 includes the mode setting unit 62, a constraint point coordinate computing unit 69, an orientation acquisition unit 67, a parameter acquisition unit 68 and a driving signal generating unit 71, which are functional units used commonly in the manual mode and the lock-on mode.

The control unit 61 includes a copy operation instruction unit 70 that is a functional unit used only in the manual mode.

The control unit 61 includes the operation amount computing unit 72, an image processing system block configured to perform processing using the image imaged by the imaging unit 5, a coordinate computing system block configured to calculate various coordinates through computation, and an inverse kinematics computing unit 82 configured to generate an operation instruction in the lock-on mode, which are functional units configured to provide a lock-on function in the lock-on mode.

The image processing system block includes an image data receiving unit 75, a feature point setting unit 76, a distance measuring unit 77 and a position-of-interest extracting unit 80.

The coordinate computing system block includes an imaging unit coordinate computing unit 79, a position-of-interest coordinate computing unit 78, a position-of-interest deviation amount computing unit 81 and a coordinate converting unit 83.

Components of the control unit 61 will be described below according to a flow of a control operation by the control unit 61.

As shown in Fig. 6, the mode setting unit 62 performs setting of an operation mode and various initial settings of the control unit 61. The mode setting unit 62 includes an initializing unit 63 and a restriction state selecting unit 64.

The initializing unit 63 receives an input of a switching signal output from the lock-on mode selecting switch 50.

The initializing unit 63 outputs a release signal for releasing all of the brakes (the first brake 46, the second brake 47 and the third brake 48) of the brake unit 45 to a brake state switching unit 66 when the input of the switching signal is received.

The initializing unit 63 resets the first sensor 41, the second sensor 42 and the third sensor 43 when the input of the switching signal is received.

The restriction state selecting unit 64 selects an operation state of each brake of the input mechanism 31 to correspond to the operation with respect to the mode selector 49. The restriction state selecting unit 64 includes a determination unit 65 and the brake state switching unit 66.

The determination unit 65 can receive the input of the signals output from the free mode switch 51, the inspection mode switch 52 and the proximity overviewing mode switch 53. In the embodiment, since the free mode switch 51, the inspection mode switch 52 and the proximity overviewing mode switch 53 are linked such that only one of them is turned ON, the determination unit 65 receives the signal from any one of the free mode switch 51, the inspection mode switch 52 and the proximity overviewing mode switch 53.

The determination unit 65 outputs a brake control pattern to the brake state switching unit 66 to correspond to a type of the signal output to the determination unit 65.

The brake state switching unit 66 receives an input of the brake control pattern output from the determination unit 65. The brake state switching unit 66 outputs a driving signal for operating the brake unit 45 according to the brake control pattern to the brake unit 45.

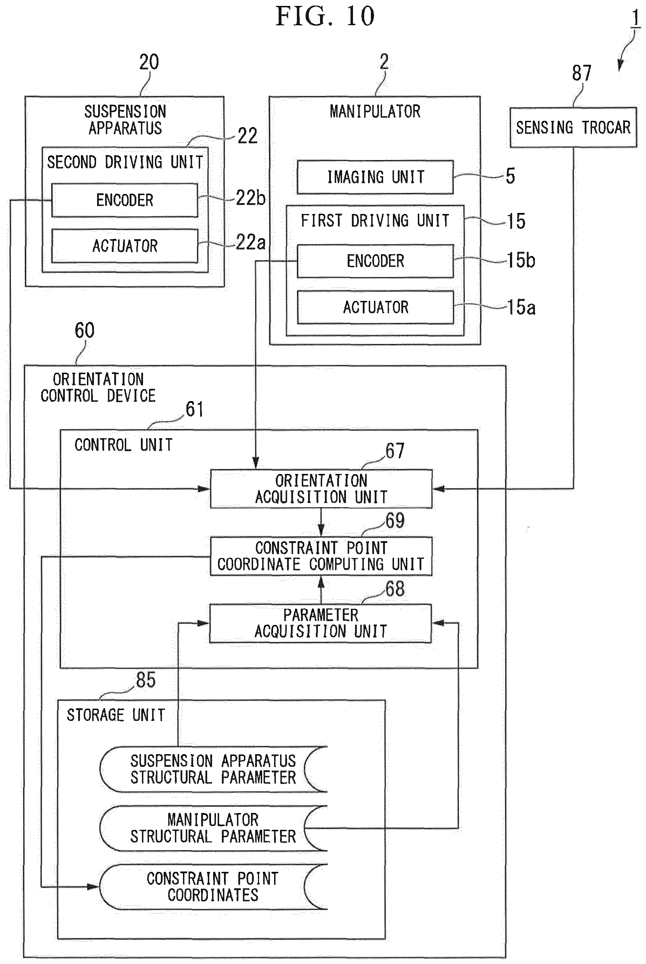

As shown in Fig. 10, the orientation acquisition unit 67 receives an input of angle information output from the encoder 15b of the first driving unit 15 of the manipulator 2. Further, the orientation acquisition unit 67 receives the input of the angle information output from the encoder 22b of the second driving unit 22 of the suspension apparatus 20.

The orientation acquisition unit 67 associates the angle information output from the encoder 15b with a predetermined identification number (a joint number) that specifies the encoders 15b. In addition, the orientation acquisition unit 67 associates the angle information output from the encoder 22b with a predetermined identification number (a joint number) that specifies the encoder 22b.

The orientation acquisition unit 67 outputs the joint number and the corresponding angle information to the constraint point coordinate computing unit 69.

The parameter acquisition unit 68 reads the suspension apparatus structural parameters and the manipulator structural parameters stored in the storage unit 85 according to a predetermined reading order.

Various parameters acquired by the parameter acquisition unit 68 are used when forward kinematics calculation or inverse kinematics calculation is performed in the control unit 61.

The constraint point coordinate computing unit 69 receives an input of the joint number and the corresponding angle information output from the orientation acquisition unit 67. Further, the constraint point coordinate computing unit 69 receives an input of shaft section position information output from the sensing trocar 87.

The constraint point coordinate computing unit 69 receives the input of the manipulator structural parameters and the suspension apparatus structural parameters output from the parameter acquisition unit 68.

The constraint point coordinate computing unit 69 calculates information of coordinates of a constraint point (constraint point coordinates) by, for example, forward kinematics calculation on the basis of the angle information and the shaft section position information. The coordinates of the constraint point are coordinates in a reference coordinate system inherent to the suspension apparatus 20.

The constraint point coordinate computing unit 69 outputs the constraint point coordinates to the storage unit 85.

The control unit 61 starts an operation in the manual mode after the constraint point coordinates are stored in the storage unit 85.

The copy operation instruction unit 70 shown in Fig. 5 is a functional unit configured to generate an operation instruction in the control unit 61 operated in the manual mode. The copy operation instruction unit 70 receives the input of the angle information and the corresponding joint number acquired by the orientation acquisition unit 67.

The copy operation instruction unit 70 generates an operation instruction for operating the joint in the same direction as the joint operation direction included in the angle information with respect to the joint relevant to the joint number corresponding to the angle information.

The copy operation instruction unit 70 outputs the operation instruction to the driving signal generating unit 71.

The driving signal generating unit 71 receives the input of the operation instruction output from the copy operation instruction unit 70. In the embodiment, when the control unit 61 is operated in the manual mode, the driving signal generating unit 71 receives the input of the operation instruction from the copy operation instruction unit 70.

The driving signal generating unit 71 receives the input of the operation instruction from the inverse kinematics computing unit 82 when the control unit 61 is operated in the lock-on mode.

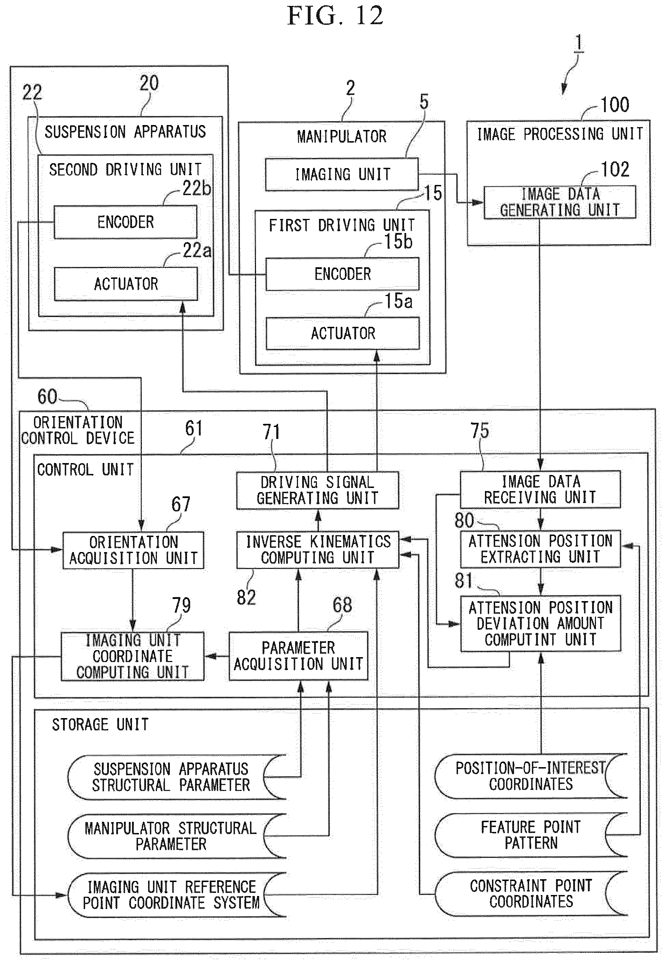

The driving signal generating unit 71 generates a driving signal with respect to the actuator 15a of the first driving unit 15 on the basis of the operation instruction. In addition, the driving signal generating unit 71 generates a driving signal with respect to the actuator 22a of the second driving unit 22 on the basis of the operation instruction, and outputs the generated driving signal to the corresponding driving unit (the first driving unit 15 and/or the second driving unit 22) (for example, see Fig. 12).

Next, components that are operated in the lock-on mode will be described.

As shown in Fig. 9, the operation amount computing unit 72 calculates an operation amount on the basis of the operation input performed on the input mechanism 31 by the operator in the lock-on mode. The operation amount computing unit 72 receives information output from the first sensor 41, the second sensor 42 and the third sensor 43. In addition, the operation amount computing unit 72 receives the input of the reset signal output from the initializing unit 63 of the mode setting unit 62 in order to reset the signals output from the first sensor 41, the second sensor 42 and the third sensor 43.

The operation amount computing unit 72 has a magnification correcting unit 73 and a forward kinematics computing unit 74.

The magnification correcting unit 73 receives the input of the information output from the third sensor 43.

The magnification correcting unit 73 calculates advance/retreat quantity information by applying a predetermined coefficient to the information output from the third sensor 43, and outputs the calculated advance/retreat quantity information to the forward kinematics computing unit 74. The coefficient defines a magnitude of an advance/retreat moving quantity of the imaging unit 5 with respect to the advance/retreat operation amount of the input member.

The forward kinematics computing unit 74 receives the input of the angle information output from the first sensor 41 and the second sensor 42, and the advance/retreat quantity information output from the magnification correcting unit 73.

The forward kinematics computing unit 74 receives the input of the manipulator structural parameter and the suspension apparatus structural parameter stored in the storage unit 85.

The forward kinematics computing unit 74 calculates information (input member position orientation data) including coordinates of the input member and an orientation of the input member by the forward kinematics calculation using the angle information, advance/retreat quantity information, the manipulator structural parameters and the suspension apparatus structural parameters. The input member position orientation data include coordinates of a predetermined point (a reference point) of the input member 39, and a direction from the reference point toward a distal part of the input member 39 (a vector in a direction from the reference point toward an origin of the input coordinate system) in a three-dimensional orthogonal coordinate system (hereinafter referred to as an input coordinate system) defined by the first axis X, the second axis Y and a vertical axis Z perpendicular to the first axis X and the second axis Y. Further, in the embodiment, when the input member 39 is disposed at a neutral position, the input member 39 extends along the vertical axis Z of the input coordinate system.

The forward kinematics computing unit 74 outputs the input member position orientation data to the storage unit 85.

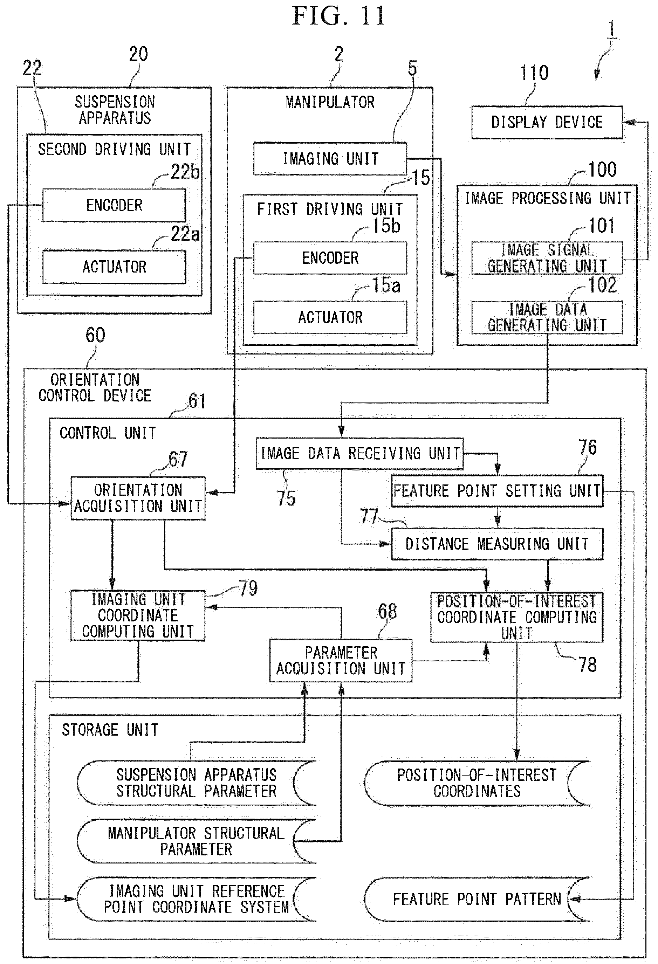

As shown in Fig. 11, the image data receiving unit 75 receives the input of the image used to be controlled in the lock-on mode. Specifically, the image data receiving unit 75 receives the input of the set of image data output from the image processing device. The image data receiving unit 75 outputs the set of image data to the feature point setting unit 76, the distance measuring unit 77, the position-of-interest extracting unit 80 and the position-of-interest deviation amount computing unit 81.

The feature point setting unit 76 receives the input of the set of image data output from the image data receiving unit 75. The feature point setting unit 76 extracts a plurality of feature points on the basis of a shape, a color, or the like in a predetermined area including a center of the first image on the basis of the first image data among the set of image data. The center of the first image in the embodiment corresponds to a position of interest Ts in the lock-on mode. The feature point setting unit 76 outputs information including feature point data showing extracted feature points and central point data showing a center of the first image, as a feature point pattern, to the storage unit 85. In addition, the feature point setting unit 76 outputs the feature point pattern to the distance measuring unit 77.

The distance measuring unit 77 receives the input of the set of image data output from the image data receiving unit 75. In addition, the distance measuring unit 77 reads the feature point pattern stored in the storage unit 85. The distance measuring unit 77 extracts a region corresponding to the feature point pattern in the second image data of the set of image data using a feature point matching method. The distance measuring unit 77 converts a magnitude of a deviation amount of a region corresponding to the feature point pattern in the first image data and the second image data to a distance value to an area in which the feature point pattern is set. In the conversion in the distance measuring unit 77, the distance measuring unit 77 acquires a distance value from the reference point previously defined in the imaging unit 5 to the area in which the feature point is set. The distance measuring unit 77 outputs the acquired distance value to the position-of-interest coordinate computing unit 78.

The position-of-interest coordinate computing unit 78 receives the input of the distance value output from the distance measuring unit 77. Further, the position-of-interest coordinate computing unit 78 receives the input of the joint number and the corresponding angle information output from the orientation acquisition unit 67.

The position-of-interest coordinate computing unit 78 receives the input of the manipulator structural parameter and the suspension apparatus structural parameters output from the parameter acquisition unit 68.

The position-of-interest coordinate computing unit 78 calculates information of coordinates (position-of-interest coordinates) of an area (a position of interest) disposed at a center in the image data by, for example, forward kinematics calculation on the basis of the angle information and the shaft section position information. The position-of-interest coordinates are coordinates in the reference coordinate system inherent to the suspension apparatus 20. The position-of-interest coordinates are coordinates of a point in front of the imaging unit 5 in an optical axis direction of the imaging unit 5 by the above-mentioned distance value.

The position-of-interest coordinate computing unit 78 outputs the position-of-interest coordinates to the storage unit 85.

The imaging unit coordinate computing unit 79 receives the input of the joint number and the corresponding angle information output from the orientation acquisition unit 67. Further, the imaging unit coordinate computing unit 79 receives the input of the manipulator structural parameters and the suspension apparatus structural parameters output from the parameter acquisition unit 68.

The imaging unit coordinate computing unit 79 calculates a bending angle of the active bending section 7 on the basis of the angle information and the manipulator structural parameters obtained from the encoder 15b of the manipulator 2. In the embodiment, the active bending section 7 is uniformly bent by pulling the angle wire w. For this reason, a bending quantity of the active bending section 7 and a center of curvature of the active bending section 7 at this time can be acquired using the angle information of the encoder 15b corresponding to the pulling amount of the angle wire w.

The imaging unit coordinate computing unit 79 calculates imaging unit reference point coordinates in the reference coordinate system using the forward kinematics calculation.

The imaging unit coordinate computing unit 79 outputs the imaging unit reference point coordinates to the storage unit 85.

As shown in Fig. 12, the position-of-interest extracting unit 80 is a functional unit configured to acquire coordinates of the position of interest from the image acquired by the imaging unit 5 during an operation in the lock-on mode.

The position-of-interest extracting unit 80 receives the input of the set of image data output from the image data receiving unit 75. In addition, the position-of-interest extracting unit 80 reads the feature point pattern from the storage unit 85.

The position-of-interest extracting unit 80 performs feature point mapping using the feature point pattern with respect to the set of image data. Accordingly, the position-of-interest extracting unit 80 extracts the feature point included in the set of image data, and calculates coordinates of a current position of the position of interest in the reference coordinate system using parallax of the set of image data.

The position-of-interest extracting unit 80 outputs the coordinate information of the current position of the position of interest to the position-of-interest deviation amount computing unit 81.

The position-of-interest deviation amount computing unit 81 receives the input of the coordinate information of the current position of the position of interest output from the position-of-interest extracting unit 80. Further, the position-of-interest deviation amount computing unit 81 reads the position-of-interest coordinates from the storage unit 85.

The position-of-interest deviation amount computing unit 81 compares the position-of-interest coordinates read from the storage unit 85 with the coordinates output from the position-of-interest extracting unit 80 and calculates a deviation amount of the position of interest. The position-of-interest deviation amount computing unit 81 outputs the deviation amount of the position of interest to the inverse kinematics computing unit 82.

The inverse kinematics computing unit 82 receives the input of the deviation amount of the position of interest output from the position-of-interest deviation amount computing unit 81. Further, the inverse kinematics computing unit 82 receives the input of the suspension apparatus structural parameters and the manipulator structural parameters output from the parameter acquisition unit 68. In addition, the inverse kinematics computing unit 82 reads the imaging unit reference point coordinates and the constraint point coordinates from the storage unit 85.

The inverse kinematics computing unit 82 calculates orientations of the manipulator and the suspension apparatus after movement by the inverse kinematics calculation from coordinates of an ending point using imaging unit reference point coordinates as a starting point and using a point obtained by moving the imaging unit 5 in parallel until a deviation amount of the position of interest becomes 0 as the ending point. In the embodiment, as the inverse kinematics computing unit 82 performs calculation such that the imaging unit 5 is moved in parallel, correspondence between the orientation of the input member and the orientation of the imaging unit 5 is maintained.

The inverse kinematics computing unit 82 generates an operation instruction for operating the manipulator 2 and the suspension apparatus 20 on the basis of the calculated result, and outputs the operation instruction to the driving signal generating unit 71.

Further, when the imaging unit 5 cannot be moved in parallel until the deviation amount of the position of interest becomes 0 due to an influence of limitation of movable ranges of the manipulator 2 and the suspension apparatus 20, an error to this effect may be output and the lock-on mode may be terminated.

Next, the functional units operated on the basis of the input performed with respect to the input mechanism 31 by the operator in the lock-on mode will be described.

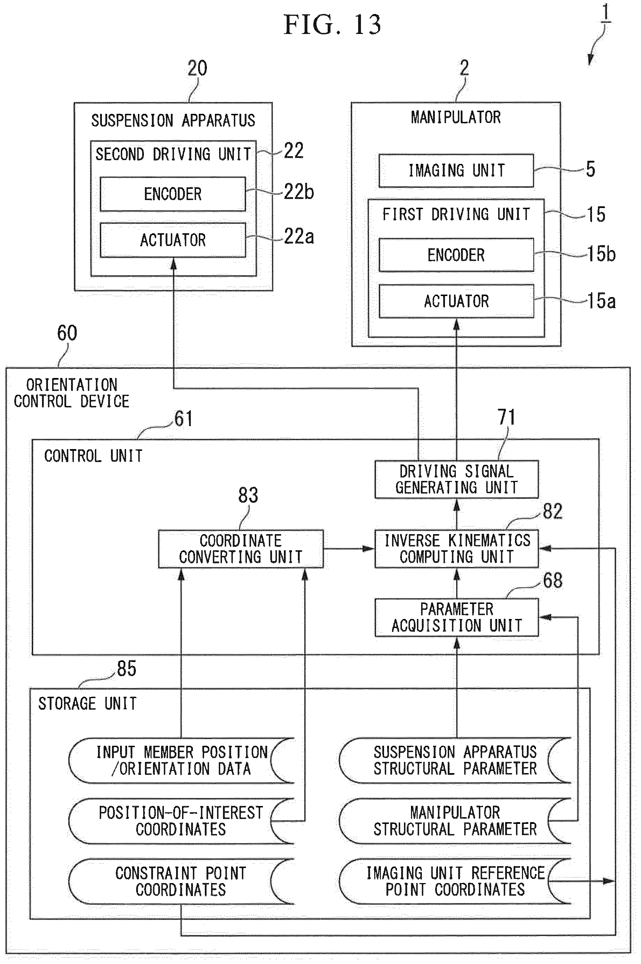

As shown in Fig. 13, the coordinate converting unit 83 reads input member position orientation data calculated by the operation amount computing unit 72 from the storage unit 85 on the basis of the operation input to the input device by the operator in the lock-on mode. Further, the coordinate converting unit 83 reads the position-of-interest coordinates from the storage unit 85.

The coordinate converting unit 83 converts the input member position orientation data into coordinates such that the position-of-interest coordinates are used as an origin. Further, the coordinate converting unit 83 moves the input member position orientation data, which are converted into coordinates, in parallel using the position-of-interest coordinates as an origin such that the origin of the reference coordinates is used as an origin. Accordingly, a position and a direction of the reference coordinate system corresponding to the input member position orientation data are calculated.

The coordinate converting unit 83 outputs the calculated position and direction to the inverse kinematics computing unit 82.

The inverse kinematics computing unit 82 receives the input of the position and the direction output from the coordinate converting unit 83. The inverse kinematics computing unit 82 that has received the input of the position and the direction output from the coordinate converting unit 83 generates an operation instruction for moving the imaging unit 5 on the basis of the position and the direction output from the coordinate converting unit 83 before the next calculation starts after the calculation on the basis of the deviation amount of the position of interest is finished.

The inverse kinematics computing unit 82 outputs the operation instruction to the driving signal generating unit 71. After that, the driving signal generating unit 71 generates a driving signal on the basis of the operation instruction and outputs the driving signal to the first driving unit 15 and the second driving unit 22.

Accordingly, the imaging unit 5 is operated according to the operation by the operator with respect to the input member of the input mechanism 31.

In the embodiment, the sensing trocar 87 is electrically connected to the control unit 61. The sensing trocar 87 outputs the information of the insertion amount of the shaft section 8 to the sensing trocar 87 with respect to the constraint point coordinate computing unit 69.

An action of the medical system 1 including the input mechanism 31 of the embodiment will be described.

Before the treatment using the medical system 1, the operator attaches the manipulator 2 of the medical system 1 to the suspension apparatus 20 (see Fig. 1). In addition, the operator disposes the suspension apparatus 20 at a predetermined position with respect to a patient, and inserts the manipulator 2 into the body. The control unit 61 of the medical system 1 stores coordinates of a constraint point R (constraint point coordinates) in the storage unit 85 as a position of an implantation point of the manipulator 2 with respect to the patient on the basis of the insertion amount of the shaft section 8 inserted into the sensing trocar 87 (see Figs. 10 and 15, step S11).

The operator moves the manipulator 2 such that a desired area (a target) serving as a treatment object is displayed in an image displayed on a monitor 111 (see Fig. 1) of the display device 110. The operator may bend the active bending section 7 using the lever 15c or the like of the first driving unit 15 according to necessity. When a target T is disposed in the vicinity of a center of the image displayed on the monitor 111 of the display device 110, the operator operates the mode selector 49 to start the lock-on mode (see Fig. 14, step S1). For example, the operator pushes the lock-on mode selecting switch 50 and the free mode switch 51.

When the control unit 61 starts to be operated in the lock-on mode (Yes in Fig. 12), the control unit 61 temporarily stops the operations of the first driving unit 15 and the second driving unit 22 (see Fig. 15, step S14). Then, the control unit 61 performs initialization and calculation required to start the lock-on mode (see Fig. 15, step S15 to step S18). First, the initializing unit 63 (see Fig. 6) of the control unit 61 outputs a release signal to the brake state switching unit 66 to release all of the brakes of the brake unit 45, and moves the input member 39 to a neutral position. Accordingly, the input mechanism 31 is initialized (step S15).

In addition, the feature point setting unit 76 (see Fig. 11) of the control unit 61 extracts the feature point from the image data and stores the feature point pattern in the storage unit 85 (step S16). Next, the position-of-interest coordinate computing unit 78 acquires the position-of-interest coordinates and stores the coordinates in the storage unit 85 on the basis of the distance value measured by the distance measuring unit 77 and the orientations of the manipulator 2 and the suspension apparatus 20 (step S17).

In addition, the imaging unit coordinate computing unit 79 (see Fig. 11) of the control unit 61 acquires imaging unit reference point coordinates and stores the coordinates in the storage unit 85 on the basis of the orientations of the manipulator 2 and the suspension apparatus 20 (step S18).

After the processing up to step S18 is finished, the operator can start the operation in the lock-on mode. When the operator grips the input member 39 (see Fig. 14, step S2), the gripping sensor detects that the operator is griping the input member 39. The operator can advance and retreat the input member 39 and swing the input member 39 while the gripping sensor detects that the input member 39 is being gripped by the operator (step S3).

As shown in Fig. 9, the operation in which the operator advances/retreats or swings the input member 39 is detected by the operation amount computing unit 72 and stored in the storage unit 85 as input member position orientation data.

As shown in Fig. 13, the coordinate converting unit 83 of the control unit 61 reads the input member position orientation data from the storage unit 85 (see Fig. 15, step S19), converts the data into the coordinates, and outputs the coordinates to the inverse kinematics computing unit 82 (step S20). Next, the inverse kinematics computing unit 82 generates an operation instruction on the basis of the information output from the coordinate converting unit 83 (step S21), and outputs the operation instruction to the driving signal generating unit 71. The driving signal generating unit 71 generates a driving signal according to the operation instruction, and outputs the driving signal to the first driving unit 15 and the second driving unit 22 (step S22).

After that, the control unit 61 corrects a position of the imaging unit 5 such that the position of interest is disposed at a center of a field of vision of the imaging unit 5 (step S23). That is, as shown in Fig. 12, the position-of-interest extracting unit 80 extracts the position of interest from the image imaged by the imaging unit 5, the position-of-interest deviation amount computing unit 81 calculates a deviation amount of the position of interest, and the inverse kinematics computing unit 82 generates an operation instruction such that a deviation amount of the position of interest becomes 0.

In this way, in the embodiment, as the movement control of the imaging unit 5 on the basis of the input with respect to the input member 39 and the movement control of the imaging unit 5 by which the position of interest is disposed at a center of the field of vision of the imaging unit 5 are sequentially performed, the imaging unit 5 revolves about the position of interest, and the center of the field of vision of the imaging unit 5 always coincides with the center of the field of vision in the lock-on mode. For example, in the lock-on mode according to the embodiment, in a state in which the imaging unit 5 captures the target T in the field of vision as shown in Fig. 16, for example, the imaging unit 5 moves in an arc shape about the target T according to the operation with respect to the input member 39. Here, the shaft section 8 of the manipulator 2 advances and retreats in the centerline direction of the shaft section 8 while swinging about the constraint point R, and further, the bent shape of the active bending section 7 is also gradually varied according to the movement of the imaging unit 5.

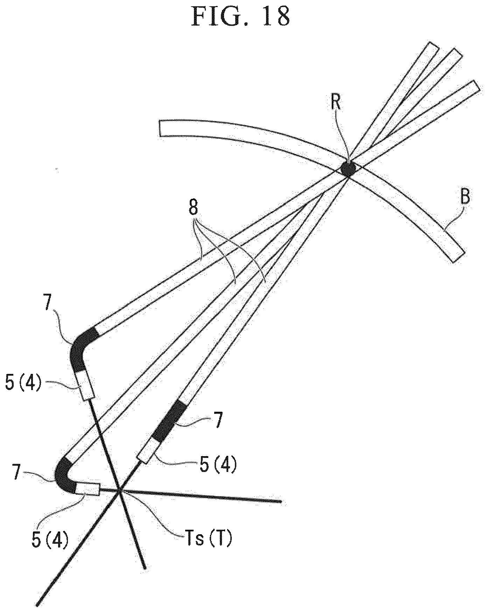

As shown in Fig. 18, in the lock-on mode of the embodiment, the imaging unit 5 can revolve about the coordinates of the position of interest Ts corresponding to the position of the target, and further, the operator can operate the distance between the position of interest Ts and the imaging unit 5 through an advance/retreat operation with respect to the input member 39. Accordingly, in the embodiment, it is possible to observe the target T by inspecting the target T, to observe an enlarged image of the target T by causing the imaging unit 5 to approach the target T, or to observe a wide region including the target T in a bird's eye view by separating the imaging unit 5 from the target T.

Steps from step S19 to step S24 shown in Fig. 15 are repeated in sequence while the lock-on mode is set (when Yes in step S24 shown in Fig. 15).

As the operator operates the mode selector 49, the operator can terminate the operation of the control unit 61 in the lock-on mode and return to the manual mode (see Fig. 15, No in step S24). In this case, automatic control of the first driving unit 15 and the second driving unit 22 is terminated at the position and the orientation of the manipulator 2 and the suspension apparatus 20 immediately before termination of the lock-on mode. After the control with respect to the first driving unit 15 and the second driving unit 22 is terminated, the brakes of the brake unit 45 are in the released state, and the input member 39 returns to the neutral position. The operator can control the position and the orientation of the manipulator 2 in the manual mode using an operation of the lever 15c of the first driving unit 15, a copy operation of the suspension apparatus 20, or the like according to necessity.

As described above, according to the input mechanism 31 of the embodiment, the operation of the input member 39 of the advance/retreat input section 37 is restricted such that the third axis L passing through the intersection between the first axis X and the second axis Y becomes an advance/retreat axis. This corresponds to restriction of the movement of the imaging unit 5 such that the imaging unit 5 is directed toward the position of interest Ts in the lock-on mode. For this reason, as the advance/retreat input section 37 is operated using the input mechanism 31 of the embodiment, an operation of changing the direction in which the imaging unit 5 approaches the position of interest Ts serving as the treatment object area in the lock-on mode can be intuitively performed.

Further, in the medical system 1 of the embodiment, since the manipulator 2 can be automatically controlled under a condition in which the position of interest Ts and the constraint point R are both restricted (see Fig. 18), in comparison with the case in which the manipulator 2 is manually operated to restrict both the position of interest Ts and the constraint point R, an operation of the manipulator 2 becomes easy.

In addition, since the first axis X and the second axis Y are perpendicular to each other, an input coordinate system CS2 in the input mechanism 31 is a three-dimensional orthogonal coordinate system. Accordingly, calculation in the control unit 61 is simple.

In addition, since the first axis X and the second axis Y are perpendicular to each other, influences applied to the operation resistance of the input member 39 by a frictional resistance around the first axis X and a frictional resistance around the second axis Y are equal to each other. For this reason, the input mechanism 31 of the embodiment can reduce variation in mobility of the input member 39 even when the position and the orientation of the input member 39 vary.

### (Variant 1)

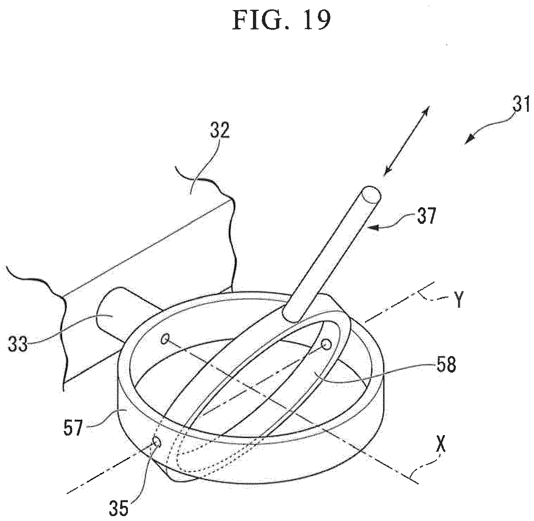

A variant of the embodiment will be described. Fig. 19 is a perspective view showing an input mechanism according to the variant.

As shown in Fig. 19, the input mechanism 31 of the variant has a first link 57 and a second link 58 having different shapes from the first link 34 and the second link 36 disclosed in the first embodiment.

The first link 57 of the variant has a ring shape having a center on the first axis X.

The second link 58 of the variant has a ring shape connected to the first link 57 on a rotation axis perpendicular to the first axis X. A center of the second link 58 is disposed on the rotation axis of the second link 58 and the first axis X. The advance/retreat input section 37 that is the same as the first embodiment is connected to the second link 58.

The first link 57 and the second link 58 of the variant is also operable similarly to the first link 34 and the second link 36 disclosed in the above-mentioned first embodiment.

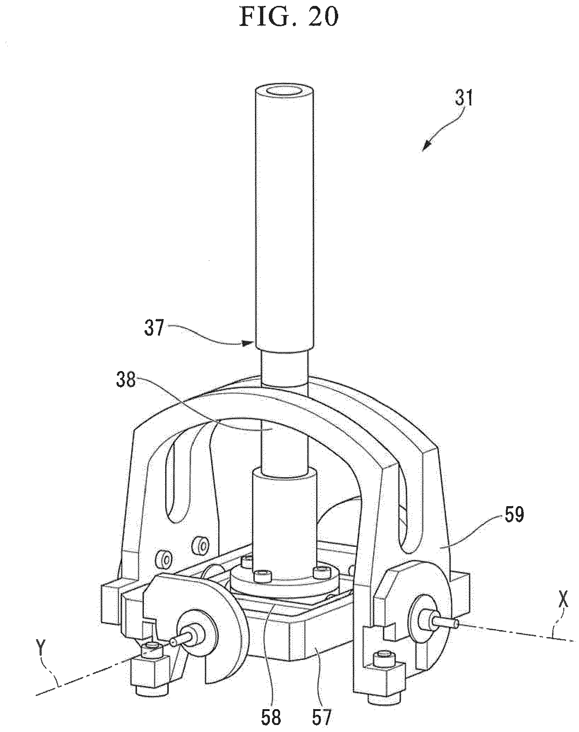

Further, as shown in Fig. 20, the shapes of the first link 57 and the second link 58 may not be a ring shape and may be a rectangular shape. In addition, the input mechanism 31 may have a guide 59 having a groove extending such that a centerline is disposed on a plane including the first axis X and attached to the first link 57. The holding section 38 of the advance/retreat input section 37 enters the groove of the guide.

### (Variant 2)

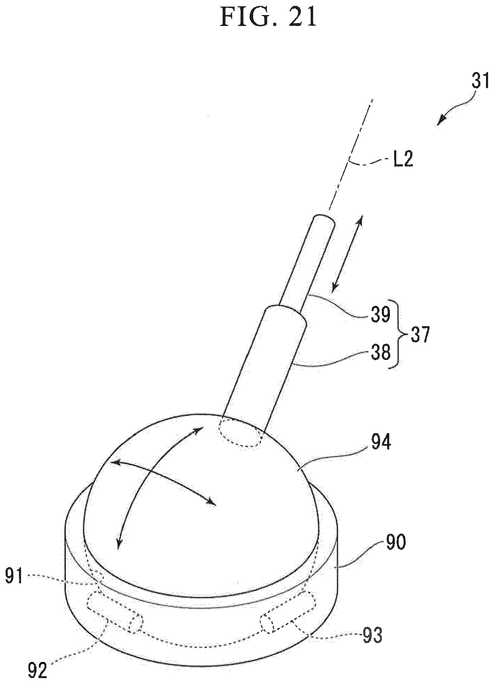

Another variant of the embodiment will be described. Fig. 21 is a perspective view showing the input mechanism 31 according to the variant.

As shown in Fig. 21, the input mechanism 31 of the variant has a base section 90, and a spherical body section 94 accommodated in the base section 90.

The base section 90 has a support body 91 configured to receive the spherical body section 94, and encoders 92 and 93 configured to come in contact with an outer circumferential surface of the spherical body section 94 and rollable. At least two encoders 92 and 93 are installed to determine the rotation direction and the rotation amount of the spherical body section.

The spherical body section 94 is accommodated in the base section 90 while being supported by the support body 91 and the encoders 92 and 93. The holding section 38 of the advance/retreat input section 37 is fixed to the spherical body section 94. A centerline L2 the holding section 38 is disposed on a straight line passing a spherical center of the spherical body section 94.

The input mechanism 31 of the variant also exhibits the same effect as the first embodiment.

### (Variant 3)

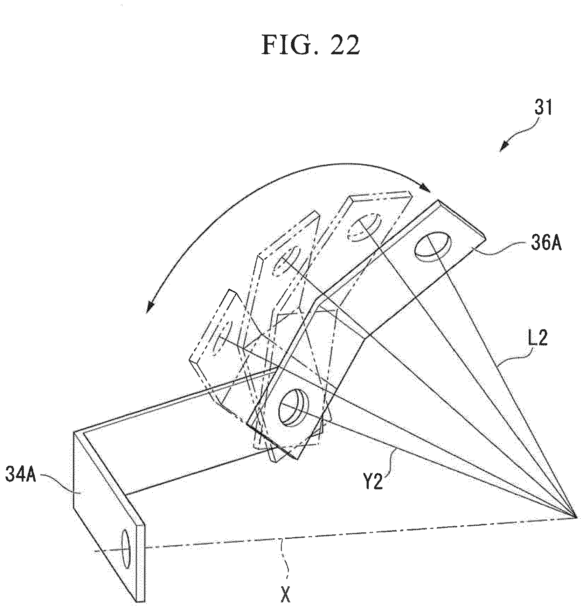

Still another variant of the embodiment will be described. Fig. 22 is a view for describing a variation in positional relation between a first link and a second link of the input mechanism of the variant.

As shown in Fig. 22, the input mechanism 31 of the variant has a first link 34A and a second link 36A having different shapes from the first link 34 and the second link 36 (see Fig. 3) disclosed in the first embodiment.

The first link 34A of the variant is bent in a "<" shape (an L shape). A bending angle of the first link 34A is an obtuse angle. For example, the first link 34A extends in a direction perpendicular to the first axis X from the first pivoting section 33 (see Fig. 3) similar to the first embodiment, and is bent by 120° to be separated from the first pivoting section 33. An end of the first link 34A opposite to an end to which the first pivoting section 33 is fixed is connected to the second pivoting section 35 (see Fig. 3) similar to that of the first embodiment.

In the variant, the second axis Y2 that is a pivot shaft of the second pivoting section 35 crosses the first axis X and is not perpendicular to the first axis X. An angle formed between the first axis X and the second axis Y2 is 60°.

The second link 36A of the variant is bent in a "<" shape (an L shape). A bending angle of the second link 36A is an obtuse angle. For example, the second link 36A extends in a direction perpendicular to the second axis Y2 from the second pivoting section 35, and is bent by 120° to approach an intersection between the first axis X and the second axis Y2. An end of the second link 36A opposite to an end to which the second pivoting section 35 is fixed is connected to the advance/retreat input section 37.

The holding section 38 (see Fig. 3) of the advance/retreat input section 37 is a tubular member about a straight line passing through an intersection between the first axis X and the second axis Y2. The input member 39 inserted through the holding section 38 can freely advance and retreat along the centerline of the holding section 38. The angle formed between the third axis L2 that is an advance/retreat axis of the input member 39 and the second axis Y2 is 60°.

In the input mechanism 31 including the first link 34A and the second link 36A of the variant, the advance/retreat operation of the input member 39 is restricted such that the third axis L2 passing through the intersection between the first axis X and the second axis Y2 is the advance/retreat axis. Further, the swing operation of the input member 39 is restricted such that the intersection between the first axis X and the second axis Y2 is a swinging center. Accordingly, the input mechanism 31 of the variant exhibits the same effect as the first embodiment.

The bending angle of the first link 34A is not limited to 120°. In addition, the bending angle of the second link 36A is not limited to 120°.

### (Second embodiment)

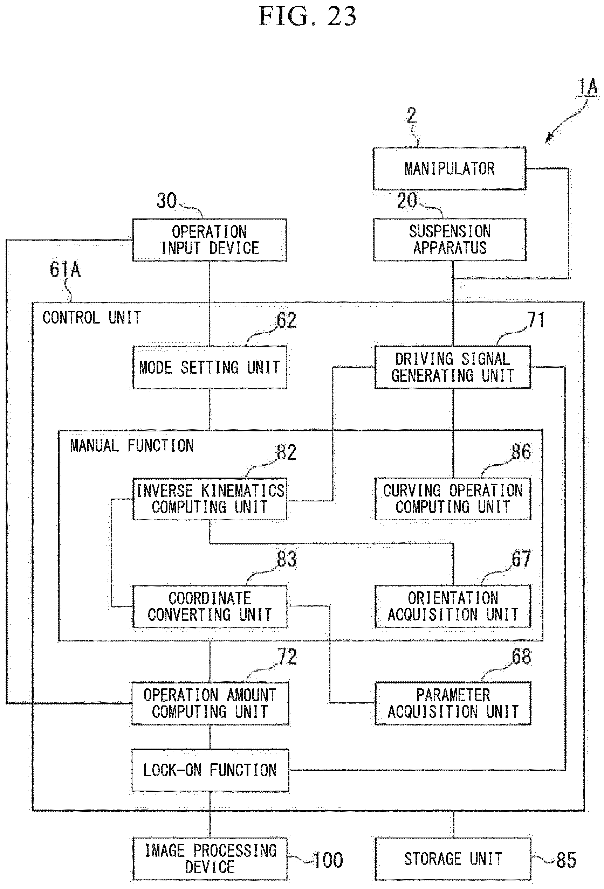

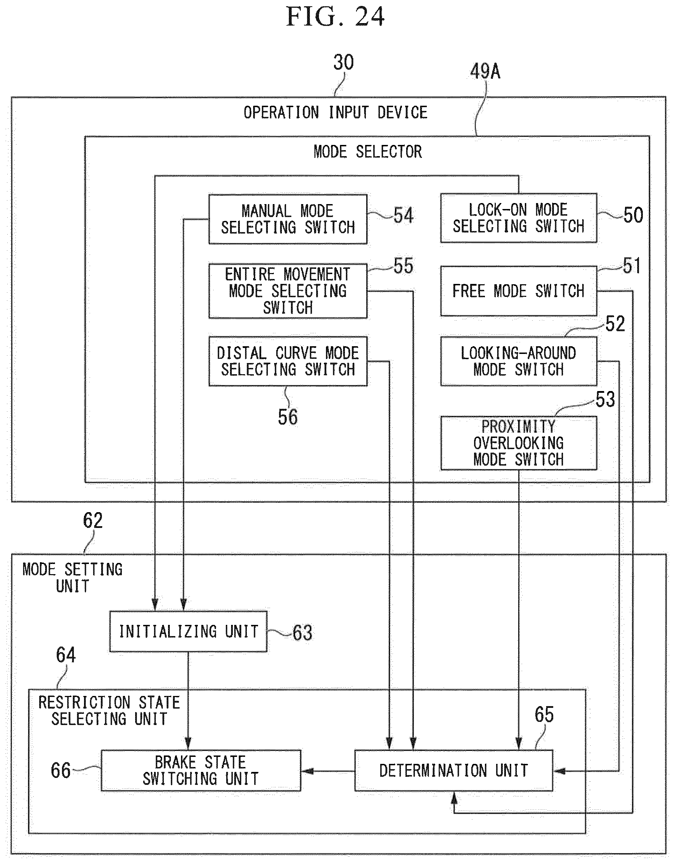

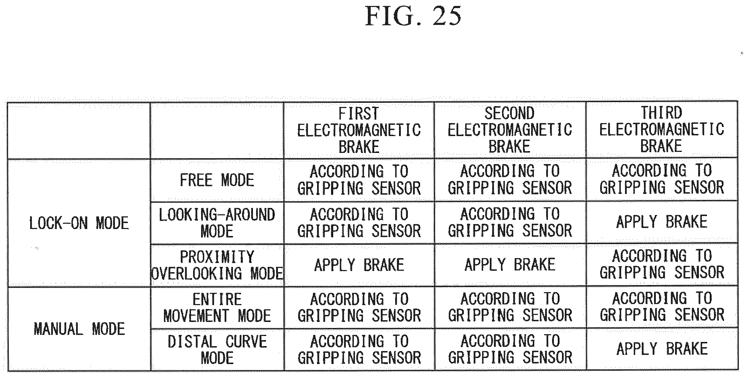

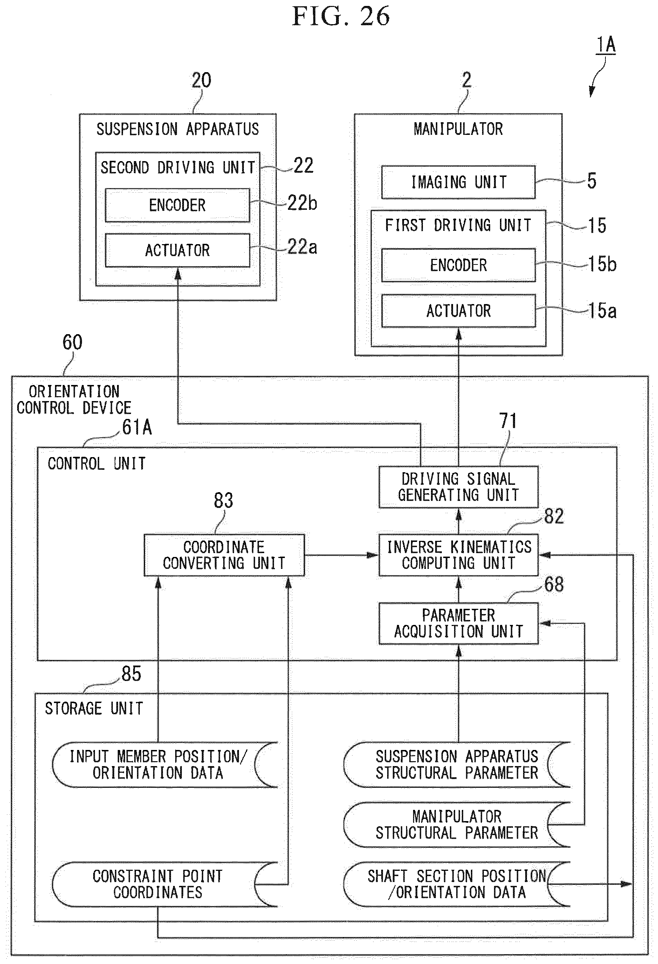

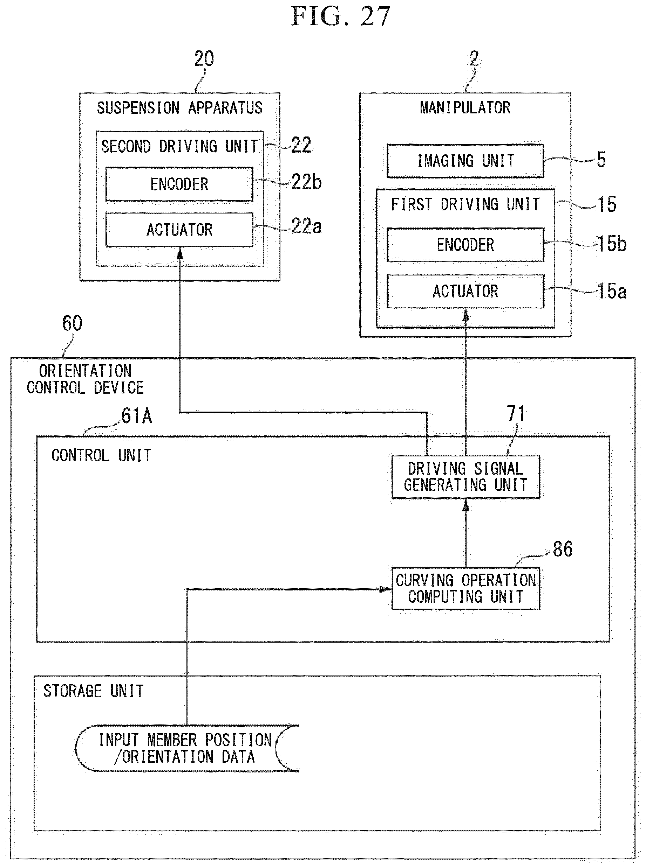

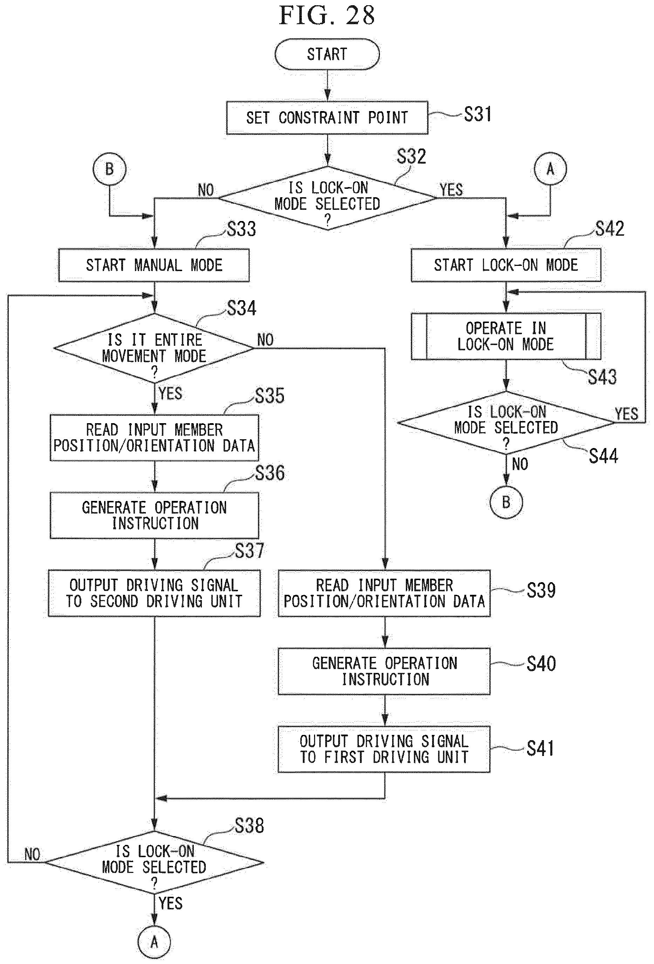

A second embodiment of the present invention will be described. Fig. 23 is a block diagram showing a major part of a medical system including an input mechanism of the embodiment. Fig. 24 is a block diagram showing a major part of the medical system. Fig. 25 is a table showing a brake control pattern of the medical system. Figs. 26 and 27 are block diagrams showing a major part of the medical system. Fig. 28 is a flowchart showing a major part of a method of actuating a medical system.

The medical system 1A of the embodiment shown in Fig. 23 has a control unit 61A having different control contents from the first embodiment. The control unit 61A of the embodiment is operable in the lock-on mode similarly to that of the first embodiment, and further, the operation input apparatus 30 can be used in the manual mode.

In addition, as shown in Fig. 24, the medical system 1A of the embodiment has a mode selector 49A having a configuration different from that of the first embodiment because the operation input apparatus 30 can be used in the manual mode.

The mode selector 49A of the embodiment is distinguished from the mode selector 49 disclosed in the first embodiment in that a manual mode selecting switch 54, the entire movement mode selecting switch 55 and a distal bending mode selecting switch 56 are further provided.

The manual mode selecting switch 54 is a push button switch that is operated when the operator selects the manual mode. In the embodiment, the manual mode selecting switch 54 and the lock-on mode selecting switch 50 are linked to each other such that when any one of them is turned ON, the other one is turned OFF.

The entire movement mode selecting switch 55 is a push button switch configured to operate when the operator selects the entire moving operation (the entire movement mode) of the manipulator 2 in the manual mode.

The distal bending mode selecting switch 56 is a push button switch configured to operate when the operator selects only a bending operation (a distal bending mode) of the active bending section 7 of the manipulator 2 in the manual mode.

In the embodiment, as shown in Fig. 25, the restriction state selecting unit 64 has a control pattern of the brake unit 45 that is usable in the manual mode.

In the entire movement mode, the operation of the brake unit 45 follows the gripping sensor like the free mode.

In the distal bending mode, only the third brake 48 is set to always apply the brake.

As shown in Fig. 26, the inverse kinematics computing unit 82 of the control unit 61A of the embodiment is configured to perform calculation on the basis of the input different from the first embodiment and output the calculated result to the driving signal generating unit 71.

Further, the orientation acquisition unit (not shown) of the control unit 61A of the embodiment calculates information (shaft section position orientation data, see Fig. 26) showing the position and the orientation of the shaft section 8 in the reference coordinate system and stores the information in the storage unit 85 using the manipulator structural parameter and the suspension apparatus structural parameter, and the information acquired from the encoders 15b and 22b.

As shown in Fig. 27, the control unit 61A further has a bending operation computing unit 86 configured to move the manipulator 2 or bend the active bending section 7 in the manual mode.

A control operation of a functional unit operated in the entire movement mode in the control unit 61A of the embodiment will be described.

In the entire movement mode, as shown in Fig. 26, the coordinate converting unit 83 reads the input member position orientation data stored in the storage unit 85, and converts the data into coordinates such that the constraint point coordinates is an origin in the reference coordinate system and outputs the coordinates to the inverse kinematics computing unit 82.

The inverse kinematics computing unit 82 reads the shaft section position orientation data from the storage unit 85. The inverse kinematics computing unit 82 sets the position and the orientation of the shaft section 8 on the basis of the shaft section position orientation data as a starting point of movement of the shaft section 8. Further, the inverse kinematics computing unit 82 sets a position and an orientation of a destination of the shaft section 8 on the basis of the information output from the coordinate converting unit 83. The inverse kinematics computing unit 82 generates an operation instruction including an operation amount of a second driving unit such that the shaft section 8 moves from a starting point to an ending point, and outputs the operation instruction to the driving signal generating unit 71. The driving signal generating unit 71 generates a driving signal according to the operation instruction and outputs the driving signal to the second driving unit.

Accordingly, in the embodiment, swing of the shaft section 8 about the constraint point and advance/retreat of the shaft section in the centerline direction of the shaft section 8 can be performed using the operation input apparatus 30.

A functional unit operated in the control unit 61A of the embodiment in the distal bending mode will be described.

As shown in Fig. 27, the bending operation computing unit 86 reads the input member position orientation data from the storage unit 85. The bending operation computing unit 86 calculates a variation in inclination of the input member from the neutral position from the input member position orientation data, and generates the operation instruction including information of a bending angle and a bending quantity of the active bending section 7 and output the operation instruction to the driving signal generating unit 71.

Accordingly, in the embodiment, a bending operation of the active bending section 7 in the manual mode can be performed using the operation input apparatus 30.

An operation of the medical system 1A of the embodiment will be described.

In the embodiment, when the lock-on mode is selected by the mode selector 49A, the medical system 1A is operated in the lock-on mode like the first embodiment.

In the embodiment, the constraint point R is set like the first embodiment (see Fig. 28, step S31).

When the manual mode is selected by the mode selector 49A in the embodiment (No in step S32), the operation in the manual mode is started (step S33), and when it is the entire movement mode (Yes in step S34), the input member position orientation data is read by the coordinate converting unit 83 and output to the inverse kinematics computing unit 82 (step S35), and the inverse kinematics computing unit 82 generates the operation instruction and output the operation instruction to the driving signal generating unit 71 (step S36). Further, the driving signal generating unit 71 outputs the driving signal to the second driving unit 22 (step S37), and the suspension apparatus 20 moves the manipulator 2 as a whole.

After step S37, when the lock-on mode is not selected by the operator (No in step S38), the processing proceeds to step S34 and continues the manual mode. After step S37, when the lock-on mode selecting switch 50 is pushed (Yes in step S38), the processing proceeds to step S42, the mode is changed from the manual mode to the lock-on mode.

In addition, after the operation in the manual mode is started, when the mode is the distal bending mode (No in step S34), the bending quantity computing unit reads the input member position orientation data (step S39), generates the operation instruction (step S40) and outputs the operation instruction to the driving signal generating unit 71. Further, the driving signal generating unit 71 outputs the driving signal to the first driving unit 15 (step S41), and the active bending section 7 is bent.

The operation (step S43) of the medical system 1A of the embodiment in the lock-on mode is similar to that of the first embodiment. In the case in which the medical system is operated in the lock-on mode, when the manual mode selecting switch 54 is pushed, selection of the lock-on mode is released. For this reason, in the case in which the manual mode selecting switch 54 is pushed when the medical system 1A is operated in the lock-on mode (No in step S44), the processing proceeds to step S33, the mode is changed from the lock-on mode to the manual mode.

The medical system 1A of the embodiment can operate the manipulator 2 using the input mechanism 31 in any one of the manual mode and the lock-on mode. For this reason, according to the medical system 1A of the embodiment, a burden of the movement or exchange when the operator operates the medical system 1A can be reduced by switching between the manual mode and the lock-on mode.

Hereinabove, while the embodiment of the present invention has been described with reference to the accompanying drawings, a specific configuration is not limited to the embodiment and may also include design changes without departing from the spirit of the present invention.

For example, the manipulator 2 may have a channel to which a known treatment tool that can be inserted into a soft endoscope can be attached. In addition, the endoscope, not limited to the treatment tool, may be inserted into the channel of the manipulator 2. When the endoscope is inserted into the channel of the manipulator 2, the imaging unit 5 of the manipulator 2 and the imaging unit 5 of the endoscope may be used in combination.

In addition, the components shown in the embodiments and the variants may be appropriately combined.

### [Industrial Applicability]

The present invention can use an input mechanism configured to perform an operation input with respect to the medical system and a medical system including the input mechanism.

### [Reference Signs List]

1 Medical system
2 Manipulator
3 Insertion section
4 Distal end portion
5 Imaging unit
6 Illumination unit
7 Active bending section
8 Shaft section
13 Insertion amount marker
14 Proximal end portion
15 First driving unit (driving unit)
15a Actuator
15b Encoder
15c Lever
16 Connecting section
20 Suspension apparatus
21 Arm section
22 Second driving unit
22a Actuator
22b Encoder
30 Operation input apparatus
31 Input mechanism
32 Base section
33 First pivoting section
34, 34A First link
35 Second pivoting section
36, 36A Second link
37 Advance/retreat input section
38 Holding section
39 Input member
40 Sensor unit
41 First sensor
42 Second sensor
43 Third sensor
44 Gripping sensor
45 Brake unit
46 First brake
47 Second brake
48 Third brake
49, 49A Mode selector
50 Lock-on mode selecting switch
51 Free mode switch
52 Mode switch
53 Proximity overviewing mode switch
54 Manual mode selecting switch
55 Entire movement mode selecting switch
56 Distal bending mode selecting switch
57 First link
58 Second link
59 Guide
60 Orientation control device
61, 61A Control unit
62 Mode setting unit
63 Initializing unit
64 Restriction state selecting unit
65 Determination unit
66 Brake state switching unit
67 Orientation acquisition unit
68 Parameter acquisition unit
69 Constraint point coordinate computing unit
70 Copy operation instruction unit
71 Driving signal generating unit
72 Operation amount computing unit
73 Magnification correcting unit
74 Forward kinematics computing unit
75 Image data receiving unit
76 Feature point setting unit
77 Distance measuring unit
78 Position-of-interest coordinate computing unit
79 Imaging unit coordinate computing unit
80 Position-of-interest extracting unit
81 Position-of-interest deviation amount computing unit
82 Inverse kinematics computing unit (operation instruction generating unit)
83 Coordinate converting unit
85 Storage unit
86 Bending operation computing unit
87 Sensing trocar
90 Base section
91 Support body
92 Encoder
93 Encoder
94 Spherical body section
100 Image processing device
110 Display device
111 Monitor
120 Light source device

## Claims

1. An input mechanism installed in a medical system having a manipulator and configured to input an operation to the manipulator, the input mechanism comprising:
a first pivoting section that is pivotable with a first axis as a rotational center;
a second pivoting section that is pivotable with respect to the first pivoting section with a second axis crossing the first axis as a rotational center;
a first link fixed to the first pivoting section and connected to the second pivoting section such that the second pivoting section is pivotable with the second axis,
a second link fixed to the second pivoting section;
an advance/retreat input section connected to the second link and thereby enable an advance/retreat operation along a third axis passing through an intersection between the first axis and the second axis as an advance/retreat axis; and
a sensor unit configured to determine a rotation angle of the first axis, a rotation angle of the second axis and an advance/retreat movement distance of the advance/retreat input section.

2. The input mechanism according to claim 1, wherein the first axis and the second axis are perpendicular to each other.

3. The input mechanism according to claim 1, wherein a first locking mechanism configured to prohibit pivotal movement with the first axis and the second axis is provided.

4. The input mechanism according to claim 1, wherein a second locking mechanism configured to prohibit an advance/retreat movement of the advance/retreat input section along the third axis is provided.

5. The input mechanism according to claim 1, wherein the first link is constituted by a rigid body bent in a "<" shape.

6. The input mechanism according to claim 1, wherein the second link is constituted by a rigid body bent in a "<" shape.

7. A medical system comprising:
the input mechanism according to claim 1;
a control unit connected to the sensor unit;
a manipulator connected to the control unit; and
a suspension apparatus configured to hold the manipulator,
wherein the manipulator has:
an imaging unit configured to image a treatment object area;
an active bending section connected to the imaging unit;
a shaft section connected to the active bending section; and
a first driving unit connected to the shaft section and configured to bend the active bending section in accordance with control by the control unit,
the suspension apparatus has:
an arm section connected to the manipulator; and
a second driving unit connected to the arm section and configured to operate the arm section in accordance with control by the control unit, and
the control unit includes:
a position-of-interest coordinate setting unit configured to set a position of interest in an imaging field of vision of the imaging unit on the basis of an image imaged by the imaging unit;
a coordinate converting unit configured to associate an input coordinate system preset in the input mechanism with a predetermined reference coordinate system such that an intersection between the first axis and the second axis corresponds to coordinates of the position of interest in the reference coordinate system, the reference coordinate system being set in the suspension apparatus in a state in which the manipulator is attached;
an operation instruction generating unit configured to allow the imaging unit to move according to a rotation angle of the first axis, a rotation angle of the second axis and an advance/retreat movement distance of the advance/retreat input section and calculate operation amounts of the arm section and the active bending section on the basis of a detection state in the sensor unit; and
a driving signal generating unit configured to operate the first driving unit and the second driving unit on the basis of the operation amount.

8. The medical system according to claim 7, wherein the control unit further comprises a constraint point coordinate computing unit configured to set a constraint point that is to be a swinging center of the manipulator in the reference coordinate system, and
the operation instruction generating unit calculates the operation amount such that the constraint point does not move in the reference coordinate system.

9. The medical system according to claim 7, further comprising:
a mode selector connected to the control unit to receive an input in order to switch between a first operation mode of operating the first driving unit and the second driving unit by using the position-of-interest coordinate setting unit, the coordinate converting unit, the operation instruction generating unit and the driving signal generating unit, and a second operation mode of allowing an operator to directly operate the manipulator.
